(19)

Europäisches
Patentamt
European
Patent Office
Office européen
des brevets

(11)  **EP 1 956 091 A2**

(12)  # EUROPEAN PATENT APPLICATION
published in accordance with Art. 158(3) EPC

(43) Date of publication:
**13.08.2008  Bulletin 2008/33**

(51) Int Cl.:
*C12N 15/12* [(2006.01)]   *C12N 15/85* [(2006.01)]
*A01K 67/027* [(2006.01)]

(21) Application number: **06841725.2**

(22) Date of filing: **01.12.2006**

(86) International application number:
**PCT/ES2006/000673**

(87) International publication number:
**WO 2007/063160 (07.06.2007 Gazette 2007/23)**

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **02.12.2005  ES 200503002**

(71) Applicants:
  • **Universidad Autónoma de Madrid
    28049 Madrid (ES)**
  • **Consejo Superior de Investigaciones Cientificas
    28006 Madrid (ES)**

(72) Inventors:
  • **VALDIVIESO ÁMATE, Fernando
    E-28049 Madrid (ES)**
  • **MONTOLIU JOSÉ, Lluís
    E-28006 Madrid (ES)**
  • **POZUETA LARIOS, Julio
    E-28049 Madrid (ES)**

(74) Representative: **ABG Patentes, S.L.
    Avenida de Burgos, 16D
    Edificio Euromor
    28036 Madrid (ES)**

(54)  **ALZHEIMER'S DISEASE ANIMAL MODEL, METHOD FOR OBTAINING SAME AND USES
THEREOF**

(57)  The invention relates to a transgenic non-human animal which can be used as a non-human animal model for studying Alzheimer's disease (AD) and which is **characterized in that**: it contains a heterologous polynucleotide (transgene) which is inserted in the genome thereof and which comprises the nucleotide sequence of the complete human APP gene together with the regulatory sequences thereof; and it has an endogenous expression pattern similar to that of the hAPP gene in humans. The inventive model can be used to study AD and in the screening of potentially-useful compounds for the prevention and/or treatment of AD.

**EP 1 956 091 A2**

**Description**

**FIELD OF THE INVENTION**

The present invention is comprised within the biotechnological and pharmaceutical sectors: the invention specifically relates to the development of transgenic non-human animals for their use as a non-human Alzheimer's disease animal model and uses thereof.

**BACKGROUND OF THE INVENTION**

In 1907, Alois Alzheimer described for the first time a neuropathological syndrome characterized by dementia and a progressive deterioration of cognitive abilities. He was also the first to demonstrate the relationship between losses in cognitive memory, neuropathological lesions observed in autopsy and the medical history of the patient. The clinical characteristics of Alzheimer's disease (AD) are a progressive loss of memory, language (aphasia), motor abilities (apraxia) and perception (agnosia), accompanied by hallucinations, depression, disorientation and aggressive behavior.

Two neurological observations carried out by Alzheimer are currently used to describe AD: (i) neurofibrillary tangles, a dense and fibrillar skein which is found inside amorphous neurons, the main subunit of which is the microtubule-associated protein Tau, and (ii) senile plaques, extracellular plaques the major component of which is a peptide called β-amyloid (Aβ) from a precursor protein called β-amyloid peptide precursor protein (APP).

The human APP gene is located in chromosome 21 in the 21q21.3 region. It is formed by 18 exons distributed throughout 300 kilobases (kb). Its expression is not limited to a specific tissue but is expressed in many cell types and tissues, including the endothelium, the glia and neurons in the brain. Messenger RNA (mRNA) can mature, experiencing an alternative method for producing three major isoforms, which are named according to the number of amino acids forming them ($APP_{770}$, $APP_{751}$ and $APP_{695}$) . The longest isoform is $APP_{770}$, containing the complete gene sequence; isoform $APP_{751}$ is the most abundantly and ubiquitously expressed isoform; and isoform $APP_{695}$ is the main isoform in the brain, the ratio between the 3 mRNAs in the cortex being $APP_{770}$:$APP_{751}$:$APP_{695}$, 1:10:20, therefore isoform $APP_{695}$ has been the most studied isoform in relation to AD.

Different mutations in the APP gene which were responsible for the development of AD were discovered between 1990 and 1992, it being possible to verify that the transmission was autosomal dominant with a penetrance of almost 100%. In 1995, two other genes related to AD were discovered, the genes called presenilin 1 (PS1) and presenilin 2 (PS2).

AD can be divided into two types based on its origin:

(i) Familial Alzheimer's disease (FAD), linked to mutations in the APP, PS1 and PS2 genes, which has a very early age of onset and is responsible for approximately 1% of Alzheimer's patients; and
(ii) Sporadic Alzheimer's disease (SAD), with a late onset (normally after 60 years of age) and an uncertain origin (it seems that the predisposition to suffer the disease is determined by a series of susceptibility genes and environmental factors, which makes it a complex and multifactorial disease, the main risk factors being old age and the existence of a family history); SAD is responsible for approximately 99% of Alzheimer's patients.

Both diseases (FAD and SAD) cannot be distinguished by the clinical symptoms and by neuropathology.

As is known, the development of experimental neurological disease models is very important for biomedical research. The development of models closely resembling the characteristics of neurodegenerative diseases in human beings has entailed a great methodological progress. In the case of AD, the main progress has been associated to identifying the proteins involved in FAD.

Several mutations linked to FAD in the APP gene were discovered at the start of the 90s decade, which, together with the progress obtained in transgenesis technology, revolutionized the possibilities of developing models for studying AD. By means of transgenesis, the pathogenic forms of the APP gene have been able to be expressed in a reproducible and more suitable manner in the different regions of the brain, thus generating a great verity of transgenic mice expressing wild APP, APP fragments, mutated APP protein and other AD-related genes.

The models developed in mice for AD include the following:

NSEAPP: a construct with the neuron-specific enolase (NSE) promoter and the APP gene isoform 751 cDNA was prepared in order to generate this mouse. The overexpression of this construct led to the occurrence of diffuse Aβ plaques at the age of 22 months. These plaques had a more mature appearance, which was reminiscent of the plaques found in Alzheimer's patients, in only 5% of the mice and were positively stained for Bielschowsky and thioflavin-S stains.

APP YAC: the use of yeast artificial chromosomes (YAC), as a carrier for generating transgenic mice, in which large amounts of DNA could be inserted, led three groups to almost simultaneously publish the generation of a transgenic mouse with a 650 kb YAC (B142F9 Washington University YAC library) including the entire APP gene encoding region and large regions at both 3' and 5' of said gene. In those mice, an expression of the human gene was achieved that was similar to that of the endogenous expression in mice, without observing the formation of plaques or neurofibrillary tangles. Human genome sequencing showed that the YAC used to generate these mice, in addition to the APP gene, contained the gene encoding the GABPA transcription factor, which could generate an unwanted phenotype.

PDAPP: this transgenic mouse, described in 1995, was the first mouse to develop a great variety of AD neuropathologies and was obtained using a construct whereby the APP gene cDNA, containing the "Indiana" V717F mutation, was overexpressed; neuritic plaques, surrounded by dystrophic neurites, astrocytosis, microgliosis and synaptic loss could be observed in the brain of the mice, but neurofibrillary tangles were never observed. The presence of plaques reached 50% occupancy in the cortical area regions, a very high percentage if it is compared to that of Alzheimer's patients, which tends to be between 6% and 12%. In spite of this large load of plaques in the brain, neurodegeneration has not been demonstrated in these mice.

Tg2576: this mouse, described in 1996, is possibly the model that has been most used to study AD; the transgene is formed by the prion protein promoter, which ensures a high expression in the brain, and with isoform $APP_{695}$ cDNA, containing the "Swedish" K670N/M671L mutation on a genetic C57BL/6J//SJL background. Tg2576 mice have human APP (hAPP) expression levels that are 5.6 times the endogenous expression levels of mice in the brain. At the age of 9-12 months, they already had neuritic plaques in the entorhinal region, later extending to the hippocampus and cortex.

APP23: this new murine model for studying AD was generated in 1997 using the Thy1 promoter and overexpressing $hAPP_{751}$ cDNA with the "Swedish" K670N/M671L mutation, whereby an expression of more than 7 times the endogenous expression was achieved, which caused the occurrence of plaques at the age of 6 months. The most important characteristic of these mice is that neuronal death in the CA1 region of the hippocampus at the age of 14-18 months was at last described. Another important characteristic of this model is the development of a cerebral amyloid angiopathy, making it a good model for studying AD. The behavioral analyses of these mice have shown that there is a wide variability in the results with significant differences in learning from the age of 18 months.

The animal models generated to date have not been able to generate all AD pathologies. The generation of these models has been based on the overexpression of the APP protein, either wild or with FAD-linked mutations, which limits such models to studying FAD, since they start from a situation which does not occur in SAD patients. The occurrence of amyloidogenic plaques in mice brains, occupying from 20 to 50% of brain regions, has been described in these models, whereas the presence of plaques does not exceed 12% in human patients.

In the model set forth herein, human APP YAC mice "hAPPy", an APP gene expression has been achieved that is very similar to the endogenous expression of mice, as described in previous works carried out with APP YAC (Buxbaum, J. D., et al. (1993). "Expression of APP in brains of transgenic mice containing the entire human APP gene." Biochem Biophys Res Commun 197 (2) : 639-45; Lamb, B. T., et al. (1993). "Introduction and expression of the 400 kilobase amyloid precursor protein gene in transgenic mice [corrected]." Nat Genet 5(1): 22-30; Pearson, B. E. and T. K. Choi (1993). "Expression of the human beta-amyloid precursor protein gene from a yeast artificial chromosome in transgenic mice." Proc Nat1 Acad Sci USA 90(22): 10578-82), with the difference that in "hAPPy" mice, the presence of the GABPA gene, which was previously in YAC, has been eliminated (Lamb, B. T., et al. (1997). "Altered metabolism of familial Alzheimer's disease-linked amyloid precursor protein variants in yeast artificial chromosome transgenic mice." Hum Mol Genet 6(9): 1535-41.). Suitable APP gene expression and possible responses in its expression due to external stimuli are essential for generating an AD model.

In spite of the efforts made to date and of having generated many AD-related neuropathologies, and even understanding FAD better, the obtained models are so far from reality that it is difficult to use them to understand the causes of SAD or the development thereof. For this reason, there is still a need to provide an AD animal model overcoming the drawbacks of existing models.

Human APP protein expression and consequently, human Aβ peptide expression make the formation of amyloid plaques possible in this model due to the fact that the murine protein cannot form them due to the differences in its sequence. The application of different environmental or pathogenic factors to "hAPPy" will thus allow a correct response of the APP gene and will allow the formation of amyloid plaques. The possibility of applying these environmental factors to the model, which factors are considered to a great extent responsible for the disease, makes it a very important tool for studying SAD.

## BRIEF DESCRIPTION OF THE DRAWINGS

Figure 1 shows the location of the APP gene in human chromosome 21 (NCBI build 35). The SeqB area excludes the entire GABPA gene encoding region but starts within its 3' UTR region.

Figure 2 shows the chromosomal location of the APP gene; A. Position of the human APP gene in chromosome 21, next to the adjacent genes noted down in ENSEMBL. B. Position of the murine APP gene in chromosome 16, next to the adjacent genes noted down in ENSEMBL.

Figure 3 shows a homology matrix of the human and murine APP gene sequences. A human chromosome 21 sequence megabase including the human APP gene is compared with a chromosome 16 megabase, in which the murine APP gene is also included.

Figure 4 shows a diagram of the PCRs of the APP gene environment. The position of the PCRs is used to delimit the size of YAC b142f9, both at 5' and at 3' of the gene, with its closest approximate distance to the gene encoding region.

Figure 5 shows a diagram indicating the demarcation of the YAC b142f9 extension by means of PCR. 1% agarose gel, with the PCRs of sequences A, B, 1 and 2. Lane 1 corresponds to the molecular weight marker. For each sequence, the negative control, YAC, and the positive control, respectively, are shown B. 1% gel, with the PCRs of sequences 3, 4, 5 and 6. Lane 1 corresponds to the molecular weight marker. For each sequence, the positive control, YAC, and the negative control, respectively, are shown.

Figure 6 shows a diagram of the steps followed in the generation and linearization of the recombination plasmid pB1R Seq 2.

Figure 7 shows a diagram of the steps followed in the generation and linearization of the recombination plasmids pYAC4 3'Seq A and pYAC4 3'Seq B.

Figure 8 shows the PCR of Exons 6 and 11 as well as of sequence 2'. PCRs of exon 6, exon 11 of the APP gene and sequence 2' of several analyzed yeast clones. M, molecular weight marker; G, genomic DNA; c+, positive control and c-, negative control.

Figure 9 shows the separation of yeast chromosomes by means of PFGE and hybridization with APP gene cDNA. A. PFGE stained with ethidium bromide. B. Membrane hybridized against APP gene cDNA. lanes: M molecular weight marker, YAC b142f9, AB1380 (YAC-free yeast), clone 13, clone 14, clone B7.

Figure 10 shows a Southern Blot of the 5' region of YAC b142f9 and of clone 14. Differences in the Southern blot band pattern between the original clone b142f9 (APP) and clone 14.

Figure 11 shows a comparative *Southern Blot* of YAC b142f9 and of clone 14. Both the DNA of clone B142F9 and of clone 14 was digested with the *Bam* HI enzyme. Separated in an agarose gel and hybridized with APP gene cDNA. M, molecular weight marker; APP, clone B142F9; 14, clone 14. On the right-hand side, B. Band pattern obtained after digesting the 300 kb of the APP gene with *Bam* HI, the fragments containing any gene encoding region are shaded.

Figure 12 shows the scheme provided for the fragmentation of YAC b142f9 by means of two successive rounds of homologous recombination in yeasts.

Figure 13 shows the dual PCR analysis of clone b106. PCR carried out on different clones, both of SeqA and of SeqB. The first PCR was carried out on exon 6 of the APP gene, whereas the second PCR was carried out on the GABPA gene. The clones positive for the first PCR and negative for the second PCR are indicated with an asterisk. C+, positive control; C-, negative control.

Figure 14 shows the PCR analysis of the recombination environment. PCR diagram, the shaded mark indicates the place of SeqB. B. PCRs of the GABPA gene, exon 11, exon 6 of the APP gene, Seq A' and Seq A. Each PCR has three lanes, corresponding to the positive control, clone b106 and negative control.

Figure 15 shows the hybridization of PFGE compared to APP gene and GABPA gene cDNA. A. PFGE gel stained with ethidium bromide. 1. Marker. 2 and 5 b142f9. 3 and 6 clone 14. 4 and 7 clone b106. B. Hybridization of lanes 1-4 with APP gene cDNA. Hybridization of lanes 5-7 with GABPA gene cDNA.

Figure 16 shows the Southern blot diagram and gel of the recombination in 3'. A. Southern blot diagram of the recombination in 3', the original YAC in the upper area and the recombined YAC in the lower area. The shaded square in front of Reco B shows the location of the probe used. B. Southern blot of the digestions with *Eco* RI *and Sca* I of the three YACs, b142f9(APP), clone 14 and clone b106.

Figure 17 shows a comparative Southern blot of clones b142f9, 14 and b106. Comparative Southern blot of YACs b142f9(APP), clone 14 and clone b106, digested with *Bam* HI and hybridized with an APP gene cDNA probe. The band pattern is conserved between the three YACs. M, marker.

Figure 18 shows the PFGE of the extraction of YAC b106. Extraction of the band corresponding to YAC b106. The sides were stained first to determine the position of the YAC in the gel. The piece of agarose corresponding to YAC b106 is missing in the central part.

Figure 19 shows a diagram of the PCRs used to analyze founder mice.

Figure 20 shows the representative PCRs of the founder mice. Sample of 23 PCRs carried out on transgenic mice, those carried out on mice 2, 12, 35, 37, 41 and 46 are shown, c- negative control, c+ positive control.

Figure 21 shows an illustrative PCR of several offspring of mouse 35 and the transmission of the APP gene to the germ line. Image of the 4 PCRs carried out on the first seven offspring of mouse 35. PCRs of *TRP* 1, exon 6, exon 11 and *LYS* 2 were carried out. It can be observed that offspring 1, 4, 5 and 7 have inherited the transgene.

Figure 22 shows a comparative Southern blot of F1 of the founder mice. Fingerprint of mice 35 and 64, compared to their non-transgenic litter siblings. A band pattern identical to that obtained for YAC b106 can be observed.

Figure 23 shows a bar chart showing human APP gene expression. Logarithmic representation in relative units of human APP gene expression in line 35. 97% confidence interval. A high expression in central and peripheral nerve tissue is detected. Lower levels in testicles, blood, heart and kidney.

Figure 24 shows a bar chart showing murine APP gene expression. Representation in relative units of murine APP gene expression in mice. The graph has been standardized against the expression in spleen (the lowest expression), using a 95% confidence interval. The legends indicate the tissue that each bar corresponds to.

Figure 25 shows the APP protein expression and assessment. A. Representative gels of the western blot analysis for APP protein and actin. Tissues: 1 cortex, 2 ventricles, 3 cerebellum, 4 mesencephalon, 5 spinal cord, 6 liver, 7 lung, 8 pancreas, 9 spleen, 10 small intestine, 11 muscle, 12 testicles, 13 heart and 14 kidney. B. Graphic representation of the APP levels. Blue bar, wild mice and red bar, transgenic mice. Tissues: 1 Cortex, 2 Ventricles, 3 Mesencephalon, 4 Cerebellum and 5 Spinal cord.

Figure 26 shows the histological analysis of transgenic mice of line 35. Congo red staining of mice brains. A Cortex of wild mice. B Hippocampus of wild mice. C Cortex of line 35. D Hippocampus of line 35. E Hippocampus of a 1-year-old Tg2576 mouse, where amyloidogenic plaques are observed.

Figure 27 shows the histological analysis of transgenic mice of line 64. Congo red staining of mice brains. A Cortex of wild mice. B Hippocampus of wild mice. C Cortex of line 64. D Hippocampus of line 64. E Hippocampus of a 1-year-old Tg2576 mouse, where amyloidogenic plaques are observed.

## DETAILED DESCRIPTION OF THE INVENTION

The present invention generally relates to a transgenic non-human animal which can be used as a non-human animal model for studying Alzheimer's disease (AD). Said transgenic animal is characterized in that it contains a heterologous polynucleotide (transgene) which is inserted in the genome thereof and which comprises the nucleotide sequence of the complete human APP gene (hAPP) together with the regulatory sequences thereof, said transgene lacking other genes present in the natural environment of the hAPP gene, such as the GABPA, ATPJ and CYYR1 genes, and in that the expression of said transgene has an endogenous expression pattern in the transgenic animal similar to the endogenous expression pattern of this same hAPP gene in humans. As used herein, the expression "endogenous expression pattern of the hAPP transgene in the transgenic animal similar to the endogenous expression pattern of the hAPP gene in humans" or the like relates to the fact that the transgenic animal containing said hAPP transgene expresses the hAPP gene in the same tissues as the hAPP gene is expressed in humans and in substantially the same proportion.

The fact that the transgenic non-human animal provided by this invention contains a transgene containing only the complete hAPP gene together with the regulatory sequences thereof, without the interference of other genes present in the natural environment of the hAPP gene (e.g., the GABPA, ATPJ and CYYR1 genes) which may interfere with its expression, allows an expression of said transgene in the transgenic animal with an endogenous expression pattern similar to the endogenous expression pattern of the hAPP gene in humans (i.e., it reproduces the situation in humans with considerable reliability), therefore said transgenic animal can be used as a non-human animal model for studying AD, its evolution and development, as well as for studying the factors and stimuli causing it and for identifying potentially useful compounds for the prevention and/or treatment of said disease.

Therefore, in one aspect, the invention relates to an isolated polynucleotide, hereinafter polynucleotide of the invention, consisting of the nucleotide sequence comprised between nucleotide 26,064,934 and nucleotide 26,642,665 of human chromosome 21 (NCBI35:21:26064934:26642665:1) or a fragment of said polynucleotide comprising the complete hAPP gene together with the regulatory sequences thereof. Said polynucleotide contains the nucleotide sequence of the complete hAPP gene together with the regulatory sequences thereof, and, flanking the ends of said nucleotide sequence of the hAPP gene, respective adjacent regions comprising unique recombination sequences, which can be used in the isolation of the nucleotide sequence of the complete hAPP gene together with the regulatory sequences thereof.

In a particular embodiment, the complete hAPP gene together with the regulatory sequences thereof is comprised between nucleotide 26,174,733 and nucleotide 26,464,809 of human chromosome 21 (NCBI35:21:26174733:26464809:1).

In another particular embodiment, the polynucleotide of the invention consists of the nucleotide sequence comprised between nucleotide 26,064,934 and the nucleotide 26,573,344 of human chromosome 21 (NCBI35:21:26064934:26573344:1). Said polynucleotide comprises the nucleotide sequence of the complete hAPP gene together with the regulatory sequences thereof flanked by regions containing at least one unique recombination sequence.

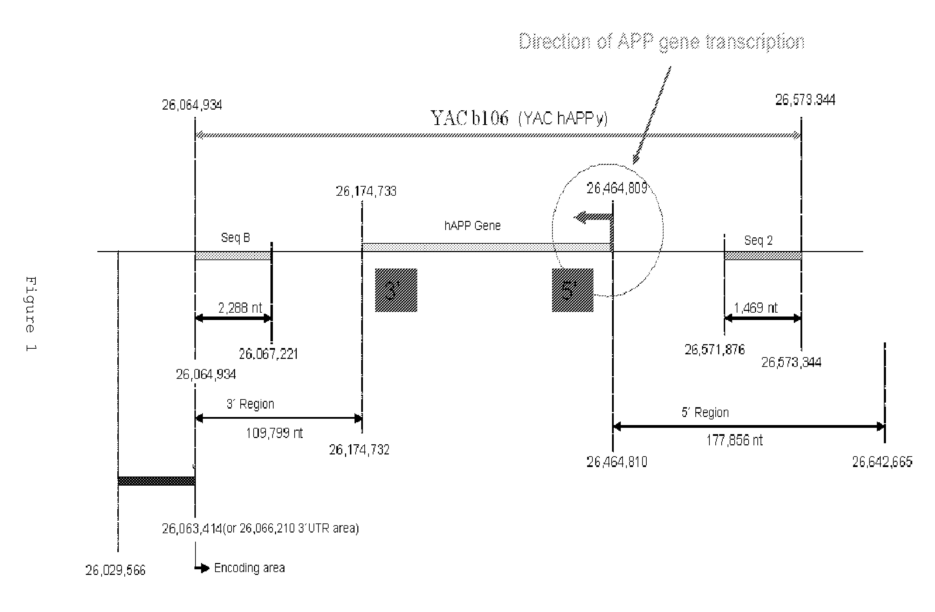

Therefore, in another particular embodiment, as shown in Figure 1, said hAPP gene is flanked at its 5' end (in relation to the origin and the direction of the APP gene transcription) by a first nucleotide sequence and at its 3' end (in relation to the end and the direction of the APP gene transcription) by a second nucleotide sequence, wherein:

- said <u>first nucleotide sequence</u> consists of the nucleotide sequence comprised between nucleotide 26,464,810 and nucleotide 26,642,665 of human chromosome 21 (NCBI35:21:26464810:26642665:1), or a fragment thereof comprising at least one unique sequence, and
- said <u>second nucleotide sequence</u> consists of the nucleotide sequence comprised between nucleotide 26,064,934 and nucleotide 26,174,732 of human chromosome 21 (NCBI35:21:26064934:26174732:1), or a fragment thereof comprising at least one unique sequence.

In a specific embodiment, said first nucleotide sequence comprises the nucleotide sequence comprised between nucleotide 26,464,810 and nucleotide 26,573,344 of human chromosome 21 (NCBI35:21:26464810:26573344:1), or a fragment thereof comprising at least one unique sequence, and said second nucleotide sequence comprises the nucleotide sequence comprised between nucleotide 26,064,934 and nucleotide 26,174,732 of human chromosome 21 (NCBI35:21:26064934:26174732:1), or a fragment thereof comprising at least one unique sequence.

The (unrepeated) unique sequences comprised in the aforementioned first and second nucleotide sequences can be used to isolate from its environment the complete hAPP gene together with the regulatory sequences thereof by means of homologous recombination, as described in the examples enclosed herein. Said unique sequences can be identified according to processes described in the state of the art and known by persons skilled in the art. By way of illustration, said processes generally include public biocomputer resources such as the RepeatMaster program [http://woody.embl-heidelberg.of/repeatmask/; Chenna Ramu *et al.,* (2000) cgi-model: CGI Programming Made Easy with Python. LINUX Journal, July, 142-149; Smit, AFA and Green, P. RepeatMasker at http://repeatmasker.genome.washington.edu/cgi-bin/RM2_req.pl); Jurka, J. 2000 Repbase Update: a database and an electronic journal of repetitive elements. Trend in Genetics 16(9): 418-420)].

In a particular embodiment, said first nucleotide sequence comprises a unique sequence comprised between nucleotide 26,571,876 and nucleotide 26,573,344 of human chromosome 21 (NCBI35:21:26571876:26573344:1), referred to as Seq 2 in the present invention.

In another particular embodiment, said second nucleotide sequence comprises a unique sequence comprised between nucleotide 26,064,934 and nucleotide 26,067,221 of human chromosome 21 (NCBI35:21:26064934:26067221:1), referred to as SeqB in the present invention.

The person skilled in the art understands that any unique sequence comprised within said first and second nucleotide sequences flanking the ends of the complete hAPP gene together with the regulatory sequences thereof can be used for the same purpose (isolating the complete hAPP gene and the regulatory sequences thereof from their environment). Therefore, the present invention is not only limited to those unique sequences described herein (forming a particular embodiment of the present invention) but also include all those unique sequences comprised within said first and second nucleotide sequences, which can be located by means of the biocomputer tools known by the persons skilled in the art and forming part of the state of the art.

The polynucleotide of the invention can be contained inside a suitable vector. Therefore, in another aspect, the invention relates to a vector, hereinafter vector of the invention, comprising a polynucleotide of the invention. Said vector therefore contains the nucleotide sequence of the complete hAPP gene together with the regulatory sequences thereof, flanked by unique sequences as described in the present invention. The choice of the vector will depend on the host cell in which it will subsequently be introduced. By way of example, the vector in which said polynucleotide is introduced can be a yeast artificial chromosome (YAC), a bacterial artificial chromosome (BAC) or a P1-derived artificial chromosome (PAC). Therefore, in a particular embodiment, the vector of the invention is selected from the group consisting of a YAC, a BAC or a PAC depending on the guest cell to be transformed.

In a particular embodiment, when the cell in which the vector of the invention is to be introduced is a yeast, the vector of choice will be a YAC, which will contain all the suitable structural elements to carry out its function, such as a telomere, a centromere, an autonomous replication sequence and an auxotrophic selection marker in each arm. The examples enclosed herein describe obtaining a YAC having the aforementioned structural elements which is used to transform yeast cells.

In another particular embodiment, when the cell in which the vector of the invention is to be introduced is a bacterium, then the vector of choice will be a BAC, in which the necessary minimum elements will be a replication origin and genes controlling the number of copies and the BAC splitting.

The characteristics of the YAC, BAC and PAC are known by the person skilled in the art. Detailed information on said types of vectors has been provided, for example, by Giraldo and Montoliú [Giraldo, P. & Montoliu L., 2001 Size matters: use of YACs, BACs and PACs in transgenic animals, Transgenic Research 10(2): 83-110].

Therefore, in a particular embodiment, the invention relates to a vector useful for transforming yeasts comprising a polynucleotide of the invention flanked at one of its ends by a third nucleotide sequence and at the other end (opposite end) by a fourth nucleotide sequence, wherein:

- said <u>third nucleotide sequence</u> comprises (i) a telomere functional in yeast cells and at least (ii) one auxotrophy selection marker, and
- said <u>fourth nucleotide sequence</u> comprises (i) a centromere functional in yeast cells, a telomere functional in yeast cells and at least one auxotrophy selection marker.

Virtually any telomere and centromere functional in yeasts can be used in the present invention. Illustrative, non-limiting examples of said telomeres and centromeres functional in yeasts include those described in the examples enclosed herein.

Likewise, virtually any auxotrophy selection marker can be used in the present invention. Illustrative, non-limiting examples of said auxotrophy selection markers include *TRP1, LEU2, LYS2, HIS3, HIS5, TRP1* and *URA3*.

The vector of the invention can be obtained by conventional methods known by persons skilled in the art [Sambrok et al., 1989 "Molecular cloning, a Laboratory Manual", 2nd ed., Cold Spring Harbor Laboratory Press, N.Y. Vol 1-3].

The vector of the invention can be used to transform eukaryotic cells, such as yeast cells, *Saccharomyces cerevisiae,* for example, or prokaryotic cells, such as bacteria, *Escherichia coli*, for example.

Therefore, in another aspect, the invention relates to a cell, hereinafter cell of the invention, comprising a polynucleotide of the invention, or a vector of the invention. The cells can be obtained by conventional methods known by persons skilled in the art [Sambrok et al., 1989 "Molecular cloning, a Laboratory Manual", 2nd ed., Cold Spring Harbor Laboratory Press, N.Y. Vol 1-3].

The described polynucleotides, vectors or cells of the invention can be used to obtain a transgenic non-human animal having, inserted in the genome thereof, the nucleotide sequence of the complete hAPP gene together with the regulatory sequences thereof.

Therefore, in another aspect, the invention relates to a transgenic non-human animal, hereinafter transgenic non-human animal of the invention, containing a polynucleotide of the invention inserted in the genome thereof. As a result, the transgenic non-human animal of the invention has an endogenous expression pattern of the hAPP gene similar to the expression pattern that this same hAPP gene has in humans. Although no link to any theory is desired, it is believed that this is mainly due to the fact that it is regulated by its own regulatory sequences. This allows the hAPP gene expression in the transgenic non-human animal of the invention to be induced by the same factors inducing its expression in humans, making it an animal model which reliably reproduces the phenomena and characteristics of AD occurring in humans.

In a particular embodiment of the invention, the transgenic non-human animal of the invention is a mammal, preferably a rodent, more preferably a mouse or a rat.

The transgenic non-human animal of the invention can have any genetic background of those known in the state of the art by a person skilled in the art, i.e., said transgenic non-human animal can come from a wild-type (wt) animal or from a non-human animal with a genetic background incorporating any molecular marker that is directly or indirectly related to AD. Therefore, in a particular embodiment, the transgenic non-human animal of the invention can express, in addition to the hAPP gene, one or more genes that are directly or indirectly related to the development of AD. Virtually any molecular marker that is directly or indirectly related to the development of AD can be used. Illustrative, non-limiting examples of said genes recognized as molecular markers of AD include presenilin 1 (PS1) gene, presenilin 2 (PS2) gene and the ε4 allele of the Apolipoprotein E (APOE) gene.

Therefore, in a particular embodiment, the genome of the transgenic non-human animal of the invention contains, in addition to a polynucleotide of the invention, one or more molecular markers of AD. By way of a non-limiting illustration, said molecular marker of AD is selected from the group consisting of the PS1, PS2 genes, and the epsilon4 (ε4) allele of the APOE gene.

Other genetic factors related to the predisposition to develop AD include the $\alpha_2$-macroglobulin gene, the very low density lipoprotein receptor gene, the gene of the lipoprotein receptor-related protein LRP, of the Tau protein, of butylcholinesterase K, of cathepsin D, of interleukin and of choline acetyltransferase.

The genetic background of the non-human mice of the present invention can additionally comprise allelic variants, mutations and polymorphisms of said molecular markers of AD.

The presence in the genome of the transgenic non-human animal of the invention of the nucleotide sequence of the complete human APP (hAPP) gene together with the regulatory sequences thereof allows said gene to be expressed with an endogenous expression pattern similar to the expression pattern that this same gene has in humans, said transgenic animal can therefore be used as a transgenic non-human animal model for studying AD. As indicated above, the transgenic non-human animal of the invention can also have, if desired, additional molecular markers of AD. All of this allows studying not only AD in general, but also different variants of Alzheimer's disease, such as FAD and SAD.

Therefore, in another aspect, the invention relates to the use of the transgenic non-human animal of the invention in studying AD. In a particular embodiment, AD is selected from FAD and SAD.

In another aspect, the invention relates to the use of a transgenic non-human animal of the invention as an AD model for studying the etiology of the disease, the causes, the triggering factors, the environmental and/or physiopathological aggressions or insults leading to the establishment of the disease.

Illustrative, non-limiting examples thereof include age, cranial trauma, female gender, the action of pathogens such as herpes type (HSV1) viruses and bacteria, bacteria, e.g., *Chlamydia pneumoniae*, etc., the accumulation of aluminium, zinc type metals in the brain, etc.

In another aspect, the invention is aimed at the use of a transgenic non-human animal of the invention for screening, discovering, identifying, evaluating and validating potentially useful compounds for the treatment and/or prevention of AD. Said compounds can be compounds of any nature, for example, a chemical, biological, microbiological compound, etc., isolated or mixed with one or more different compounds, and includes compounds with a known or unknown composition and structure, pharmaceutical products with any known therapeutic application, biological products, microbiological products, etc., for example, organic or inorganic chemical compounds, peptides, proteins, nucleic acids, extracts, etc.

The transgenic non-human animal of the invention, e.g., a mouse, can additionally be used by means of crossing it with other non-human animals of the same species to obtain non-human animals having the genotype and phenotype characteristics of their progenitors. Examples of non-human animals which can be crossed with the non-human animal of the invention include:

- knock-out non-human animals for the APP gene, or other genes directly or indirectly related to AD;
- non-human animals expressing allelic variations, polymorphisms or mutations in genes involved in AD, or
- other non-human animals which can be used as AD models.

In a particular embodiment, the genes related directly or indirectly to AD are selected from the group consisting of the PS1, PS2 genes, and the epsilon4 ($\varepsilon$4) allele of the APOE gene, or other genetic predisposition factors related to the disease, such as the $\alpha_2$-macroglobulin gene, the very low density lipoprotein receptor gene, the gene of the lipoprotein receptor-related protein LRP, of the Tau protein, of butylcholinesterase K, of cathepsin D, of interleukin and of choline acetyltransferase.

In another particular embodiment, the transgenic non-human mouse of the invention can be crossed with a knock-out mouse for the murine APP (mAPP) gene, such that after several crossings, a mouse expressing only the hAPP transgene can be obtained.

Therefore, in another aspect, the invention relates to the use of a transgenic non-human animal according to the invention to obtain a transgenic non-human animal expressing only the hAPP transgene and not the mAPP gene.

In another aspect, the invention relates to a method for identifying a potentially useful compound for the treatment and/or prevention of AD, comprising:

a) administering a candidate compound to a transgenic non-human animal of the invention;
b) determining the expression pattern of the hAPP gene and/or the hAPP protein production and/or the $\beta$-amyloid (A$\beta$) peptide production in said transgenic non-human animal of step (a), and
c) selecting the candidate compound causing (i) a decrease in the hAPP gene expression levels in nervous tissue, (ii) a decrease of hAPP protein production and/or accumulation and/or (iii) a decrease in A$\beta$ peptide production and/or accumulation in said transgenic non-human animal.

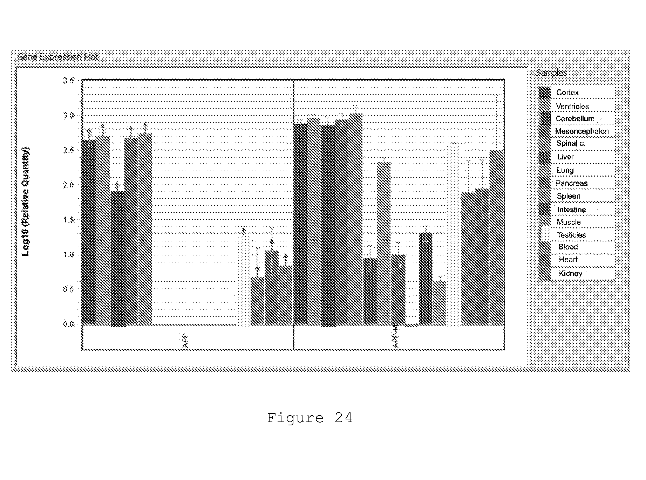

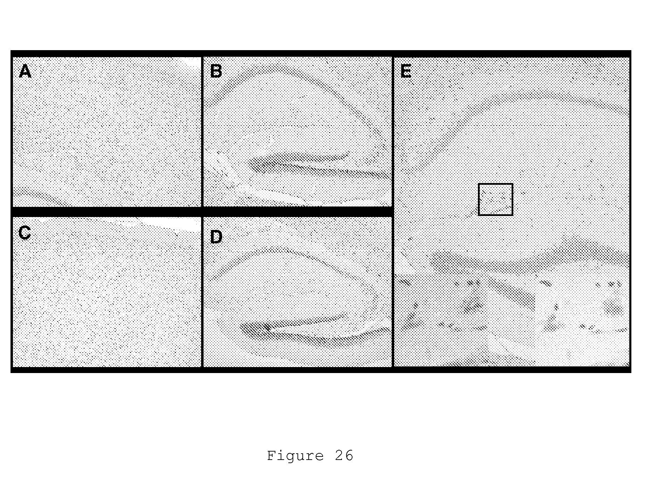

The hAPP gene expression levels, the hAPP protein production and/or accumulation levels as well as the A$\beta$ peptide production and/or accumulation levels in nervous tissue can be measured by means of conventional techniques known by the person skilled in the art, such as quantitative RT-PCR, Northern blot, Western blot, etc. Preferred ranges of hAPP gene expression levels or of hAPP protein accumulation levels can be found in Figures 24 and 26 respectively.

In a particular embodiment, the candidate compound causing a decrease in the hAPP gene expression levels, or a decrease in the hAPP protein production and/or accumulation levels or a decrease in A$\beta$ peptide production and/or accumulation compared to the control levels is selected. Said compound can be any of the compounds mentioned above.

The transgenic non-human animals of the invention can be obtained by any transgenesis method known by the person skilled in the art, such as microinjection, electroporation, particle bombardment, cell transformation followed by cloning (the nuclei of the successfully transformed cells are transferred to enucleated ova and are implanted in receptor females), gamete transformation (introducing genes in oocytes or spermatocytes and using the transformed gametes for fertilization, generating a complete animal) and/or intracytoplasmic sperm microinjection (ICSI).

In a particular embodiment, as described in the examples enclosed herein, the transgenesis method used is based on intracytoplasmic sperm microinjection. Said technique is extensively described in international patent application WO 2005/098010.

The animals obtained by the previously described transgenesis method have the characteristic genotype and phenotype of the transgenic non-human animal of the invention, they contain, inserted in their genome, the nucleotide sequence of the complete hAPP gene together with the regulatory sequences thereof and said hAPP gene has an endogenous expression pattern similar to the expression pattern that this same gene has in humans.

Like the transgenic non-human animal of the invention, the transgenic non-human animals obtained by the previously described transgenesis method can also be crossed with other non-human animals of the same species to obtain transgenic non-human animals having the genotype and phenotype characteristics of their progenitors, giving rise to different non-human animals which can used as a model for studying AD.

In addition, the descendants of the transgenic non-human animals obtained by the transgenesis method described in the present invention can have an endogenous expression pattern of the hAPP gene similar to the pattern of said protein in humans.

Another aspect of the present invention therefore relates to the offspring of a transgenic non-human animal, which can be obtained according to the transgenesis method described in the invention, endogenously expressing the human APP gene with an expression pattern similar or equivalent to the expression pattern of said gene in humans.

The following example illustrates the invention and must not be considered in a limiting sense thereof.

**EXAMPLE**

**Obtaining a transgenic non-human animal endogenously expressing the complete human APP gene with an expression pattern equivalent to the expression pattern of said gene in humans**

**MATERIAL AND METHODS**

**1. Plasmid Generation**

1.1 General Cloning

The usual molecular biology techniques (Sambroock, J., 1989, mentioned above; Ausubel FM, B. R., Kingston RE, Moore DD, Seidman JG, Smith JA and Struhl, K. (1999). Current Protocols in Molecular Biology, John Wiley and Sons.) and techniques refined in the laboratory of the inventors (Montoliu, 1997 Lab protocols. Generation of a transgenic mouse) were used to generate the necessary vectors for this work.

1.2 DNA Electrophoresis

The separation, identification and quantification of the different DNA fragments were carried out by means of electrophoresis in agarose gels. Ultra Pure Agarose (GIBCO-BRL) at concentrations comprised between 0.5 and 2% was used, a range of molecules from 100 bp to 12 kb being able to be separated. Horizontal Ecogen cells (6.5 x 8 cm and 11 x 15 cm) were used in which a current of 5 V/cm was applied with a Segainvex electric source. The gels were made in TAE buffer (40 mM Tris-Acetate, 2 mM EDTA (ethylenediamine tetraacetic acid) pH 8 (Merck)) and with ethidium bromide (Roche) at 0.5 $\mu$g/ml.

1.3 DNA Quantification

DNA quantification was carried out by means of different methods, depending on the necessary precision and the expected concentration. The estimation of the DNA concentration in samples the expected concentration range of which ranged between 0.05 and 2 $\mu$g/$\mu$l was carried out in the SHIMADZU UV-1601 model spectrophotometer, based on the spectrophotometric measurement of the DNA absorbance at a wavelength of 260 nm. The measurements were carried out according to the instructions of the company and taking into account that one unit of optical density at a wavelength of 260 nm ($OD_{260}$) corresponds to 50 $\mu$g/ml of double-stranded DNA, the DNA concentration was calculated according to the following equivalence:

$$\text{Double-stranded DNA concentration in } \mu g/ml = OD_{260} \, x \, \frac{50 \mu g/ml \; x \; Dilution\, Factor}{1 OD_{260}}$$

The Hoefer DyNA Quant 200 Fluorometer (Amersham Pharmacia Biotech) was used to estimate samples the concentration of which was estimated between 0.01 and 0.05 $\mu$g/$\mu$l. This method measures the fluorescence of bisbenzamide (Hoeschst 33258), which molecule has the capacity to bind to the double-stranded DNA, emitting fluorescence which can be detected by the fluorometer when it is excited by means of UV light at 365 nm. This method is very useful to measure in small concentration ranges, because the background due to loose nucleotides or RNA is prevented. All these measurements were verified by means of nanodrop (NanoDrop, ND-1000 spectrophotometer).

1.4 <u>Polymerase chain amplification reaction (PCR)</u>

DNA fragments for cloning and the amplifications of specific YAC regions, both for analyzing recombinations and for verifying the integration thereof in transgenic mice, were obtained by means of PCR.

PCR requires specific conditions for each amplified fragment. The mixture reaction concentrations, like the temperatures, amplification times and cycles, vary for the different reactions. The standard starting conditions for all the reactions are as follows: 1.5 mM MgCl$_2$ (Biotools), 200 $\mu$M deoxynucleotides [dATP, dCTP, dTTP and dGTP (Roche)], specific pair of oligonucleotides at 1 $\mu$M (Isogen), 1x reaction buffer (Biotools) and 1 unit of Taq polymerase (Biotools), all in a volume of 25 $\mu$l. The reaction was carried out in 0.2 ml tubes (MJ Research) in the MJ Research PCT-100 model thermal cycler. The programs used varied depending on the fragment to be amplified. They generally consisted of a first DNA denaturation at 95°C for 5 minutes, then a cycle of 3 temperatures which was repeated 35 times, 30 seconds at 95°C, 30 seconds between 55-65°C depending on the oligonucleotide hybridization temperature and 1 minute at 72°C. They were finally subjected to 10 minutes at 72°C for the complete amplification of the fragments.

1.4.1 PCR of yeast colonies

The yeast colonies were analyzed to study the different homologous recombinations. In order to carry out a direct PCR of the colony, preventing growing them and extracting the DNA therefrom, the colony was transferred to a PCR tube (0.2 ml) in which it was resuspended in 50 $\mu$l of Zymolyase (20 U/ml), incubating for 10 minutes at room temperature. The tubes were centrifuged at 5,000 r.p.m. in an Eppendorf 5417c table top centrifuge, the supernatants were eliminated and the cells were heated for 5 minutes at 95°C. The precipitate was resuspended in 15 $\mu$l of water and 5 $\mu$l were used to carry out the PCR.

1.5 <u>DNA purification and extraction from agarose gels</u>

The DNA fragments of less than 10 kb were purified by means of agarose gels in TAE buffer. After separating the fragments in the gel, they were identified by means of a UV lamp (Upland). The bands were then cut with a sterile blade and were introduced in an Eppendorf tube. The DNA was extracted from the gel by means of the QIAagen Qiaquick gel extraction kit according to the instructions described in the protocol thereof. 1.6 <u>Prokaryotic Cells</u>

1.6.1 Bacterial Strains and Culturing

The recombinant plasmids were amplified in commercial E. coli strains. The plasmids the size of which was about 5 kb were amplified and transformed into DH5$\alpha$ cells (Gibco), whereas the plasmids with a size close to 10 kb were amplified and transformed into TOP10F' cells (Stratagen).

The bacterial growth for the amplification of the different plasmids was carried out in LB (Luria-Bertani) liquid medium at 37°C for at least 8 hours with stirring (250 r.p.m.) and in the presence of ampicillin (Sigma), at a final concentration of 50 $\mu$g/ml. Colony screening was carried out after growing the transformations in Petri dishes with agar and LB medium in the presence of ampicillin and, in the cases in which color screening could be carried out, with X-gal and IPTG.

1.6.2 Preparation and transformation of competent bacteria

Competent DH5$\alpha$ cells were prepared by means of the calcium chloride method and were transformed by means of the thermal shock method.

Large plasmids were transformed using commercial TOP10F' strains (Stratagen). Electroporation was carried out in electroporation cells with a diameter of 2 mm (Bio Rad) previously cooled in ice, and giving a pulse in conditions of 25 $\mu$F, 2.5 KV and 200 $\Omega$ using the Bio-Rad Gene Pulser according to the indications of the company.

1.6.3 Plasmid DNA purification

Small-scale plasmid DNA purifications were carried out inoculating the colony in question in 2 ml of LB medium and antibiotics. These cultures were grown for at least 8 hours at 37°C and with stirring (250 r.p.m.). The plasmid DNA was extracted using the alkaline lysis method. The preparation of plasmid DNA at a greater concentration and amount was carried out by means of culturing large amounts of bacteria (100-500 ml) using commercial Qiagen maxi kits (Qiagen) and Flexiprep Kits (Amersham Pharmacia) according to the specific protocols of each kit.

## 2. Oligonucleotides

The oligonucleotides used to delimit the flanking 5' and 3' regions of the APP gene in the YAC, and their distance to the closest encoding gene region, are:

APP3'-B-U 5'-atcctaagaggaagggatcttaagg-3' [SEQ ID NO: 1]
APP3'-B-L 5'-gggagaacaaacatacctcactagc-3' [SEQ ID NO: 2]
APP3'-A-U 5'-ccagcatgttttccaaagtatgag-3' [SEQ ID NO: 3]
APP3'-A-L 5'-tgaaatgtccccacactgatcactg-3' [SEQ ID NO: 4]
APP3'-1-U 5'-gtatagtatggatggttcaaacagagccta-3' [SEQ ID NO: 5]
APP3'-1-L 5'-aaggtaaagctctagaatctatcagtgcct-3' [SEQ ID NO: 6]
APP3'-2-U 5'-tttagaagagctcagtaagaatccacattt-3' [SEQ ID NO: 7]
APP3'-2-L 5'-cattttgtcgttactagtaccattggtttc-3' [SEQ ID NO: 8]
APP5'-1-U 5'-ctcaaaaacagcaacacagatgtgc-3' [SEQ ID NO: 9]
APP5'-1-L 5'-gagcttaaacaccttcctctgc-3' [SEQ ID NO: 10]
APP5'-2-U 5'-tggggagaggaatggaatttggagc-3' [SEQ ID NO: 11]
APP5'-2-L 5'-gtcatttccagaaaaagcccactgc-3' [SEQ ID NO: 12]
APP5'-3-U 5'-tcaaggacatggagattgggcaggc-3' [SEQ ID NO: 13]
APP5'-3-L 5'-ggggtaacaggttgccggtcatatg-3' [SEQ ID NO: 14]
APP5'-4-U 5'-caccagaccatgatgtctcagtagc-3' [SEQ ID NO: 15]
APP5'-4-L 5'-ctgcaaaacagccctcatattctgc-3' [SEQ ID NO: 16]
APP5'-5-U 5'-tagcatcctttgctaagccagttgc-3' [SEQ ID NO: 17]
APP5'-5-L 5'-gcttgttattggaggttccagcacg-3' [SEQ ID NO: 18]
APP5'-6-U 5'-tccaacgggggagtgagtgaaaggc-3' [SEQ ID NO: 19]
APP5'-6-L 5'-gcctcactcctgcaaacgtgcccaa-3' [SEQ ID NO: 20]
APP5'-7-U 5'-tctttcttccaccttggtatcctgc-3' [SEQ ID NO: 21]
APP5'-7-L 5'-caggatgtctctggatttttactcg-3' [SEQ ID NO: 22]

The oligonucleotides used to verify the exons of the APP gene, as well as the presence of the GABPA gene, are the following:

APP Ex1-U 5'-agtttcctcggcagcggtagg-3'[SEQ ID NO: 23]
APP Ex1-L 5'-ccagcaggagcagtgccaaac-3'[SEQ ID NO: 24]
APP-EX2-U 5'-aagaccgggctgattcctaa-3'[SEQ ID NO: 25]
APP-EX2-L 5'-tccaacgtgaattgctagcc-3'[SEQ ID NO: 26]
APP-EX3-U 5'-cccaagcattttggataagg-3'[SEQ ID NO: 27]
APP-EX3-L 5'-cctctttttcttccctcaag-3'[SEQ ID NO: 28]
APP-EX4-U 5'-ttgattgggttgcttaggca-3'[SEQ ID NO: 29]
APP-EX4-L 5'-tgttgcctcaaaatacccct-3'[SEQ ID NO: 30]
APP-EX5-U 5'-ctaccactcactgttttctc-3'[SEQ ID NO: 31]
APP-EX5-L 5'-gcagagacctttttcagtgat-3'[SEQ ID NO: 32]
APP-EX6-U 5'-tgccaaaattccatatggacg-3'[SEQ ID NO: 33]
APP-EX6-L 5'-gggatttgccaagcagcatat-3'[SEQ ID NO: 34]
APP-EX7-U 5'-ccactgggaggattaaaaga-3'[SEQ ID NO: 35]
APP-EX7-L 5'-gagaagtggacagaaatgtg-3'[SEQ ID NO: 36]
APP-EX8-U 5'-tgtcagtggactcgtgcatt-3'[SEQ ID NO: 37]
APP-EX8-L 5'-catctcaagctgtctggcaa-3'[SEQ ID NO: 38]
APP-EX9-U 5'-catgtcttcagcaccaactg-3'[SEQ ID NO: 39]
APP-EX9-L 5'-caaactgtgcccacacagta-3'[SEQ ID NO: 40]
APP-Ex10-U 5'-cagataggaaggggtatgta-3'[SEQ ID NO: 41]

APP-Ex10-L 5'-ggagcaaatataaggcagga-3'[SEQ ID NO: 42]
APP-EX11-U 5'-tgatgagggttggagagtgca-3'[SEQ ID NO: 43]
APP-EX11-L 5'-caagatggaatggacaggggt-3'[SEQ ID NO: 44]
APPEx12-13-U 5'-cctcgtcacgtgttcaatat-3'[SEQ ID NO: 45]
APPEx12-13-L 5'-caacttcatcctgaatctcc-3'[SEQ ID NO: 46]
APPEx14-U 5'-cacgtgaaagcagttgaagt-3'[SEQ ID NO: 47]
APPEx14-L 5'-ttgccacctatacaatggag-3'[SEQ ID NO: 48]
APPEx15-U 5'-ctggcacatcaatagcgata-3'[SEQ ID NO: 49]
APPEx15-L 5'-actcggaacttgggaaatga-3'[SEQ ID NO: 50]
APPEx16-U 5'-taaaggcagcagaagcctta-3'[SEQ ID NO: 51]
APPEx16-L 5'-gctcagcctagcctatttat-3'[SEQ ID NO: 52]
APPEx17-U 5'-accagttgggcagagaatat-3'[SEQ ID NO: 53]
APPEx17-L 5'-cttgagcagaatattcacgg-3'[SEQ ID NO: 54]
APPEx18-U 5'-cttggtaattgaagaccagc-3'[SEQ ID NO: 55]
APPEx18-L 5'-cttttgacagctgtgctgta-3'[SEQ ID NO: 56]
GABPA-EX-U 5'-tattattacgatggggacat-3'[SEQ ID NO: 57]
GABPA-EX-L 5'-gtaaagcaacatctaaagca-3'[SEQ ID NO: 58]

The oligonucleotides used for the sequencing of the different cloning vectors used for the recombination are:

SV40A-2 5'-gtgaaggaaccttacttctgtggtg-3'[SEQ ID NO: 59]
Sp6 Promoter 5'-atttaggtgacactata-3'[SEQ ID NO: 60]
T7 Promoter 5'-taatacgactcactataggg-3'[SEQ ID NO: 61]

The oligonucleotides used to clone the yeast URA gene are:

URA3-U 5'-gcccagtattcttaacccaaactgca-3'[SEQ ID NO: 62]
URA3-L 5'-cttccacccatgtctctttgagcaa-3'[SEQ ID NO: 63]

The oligonucleotides used to detect auxotrophic YAC sequences are:

TRP1 5'-gcccaatagaaagagaacaattgacc-3'[SEQ ID NO: 64]
TRP2 5'-acacctccgcttacatcaacacc-3'[SEQ ID NO: 65]
LYS1 5'-accaagccagcatctgtatcacc-3'[SEQ ID NO: 66]
LYS2 5'-gccaaatccatccacttctcatc-3[SEQ ID NO: 67]

The oligonucleotides used to generate probes for the Southern blot analysis are:

ProbeAPP-seq2-U 5'-tgaatcttggcatttgggcc-3'[SEQ ID NO: 68]
ProbeAPP-seq2-L 5'-cctgtaacttgccctcatag-3'[SEQ ID NO: 69]
ProbeAPP-seqA-U 5'-gaaacgagttgaaaggcacag-3'[SEQ ID NO: 70]
ProbeAPP-seqA-L 5'-tttacccttccatgaagtccc-3'[SEQ ID NO: 71]
ProbeAPP-seqB-U 5'-ggcttgtaaattaaactctgaagc-3' [SEQ ID NO: 72]
ProbeAPP-seqB-L 5'-cctcagtggctgttttgagagcat-3'[SEQ ID NO: 73]

### 3. Yeast artificial chromosome modifications

3.1 Eukaryotic cells

All the experiments carried out in yeasts were carried out in the *S. cerevisiae* AB1380 strain the metabolic characteristics of which are: *Mata, ura3-52, trp1, ade2-1, lyes2-1, his5 and can1-100.* The original clone used was purchased in *Washington University, Human Yeast Artificial Chromosome Clone,* which contained the YAC B142F9 (650 Kb YAC containing the complete human APP gene).
The YAC carrier yeasts were grown in liquid and solid medium with autotrophic selectors to prevent the sporadic YAC elimination. The liquid cultures were grown for 2-3 days at 30°C and with stirring (200 r.p.m.), whereas the yeasts were grown for 4-5 days at 30°C to obtain colonies in plates.
The selective media were prepared with D+Glucose (Merck), YNB (*Yeast Nitrogen Base w/o Amino Acids*) (Difco) and the corresponding amino acids (Serva).

3.2 Homologous recombination in yeasts

The transformation of yeast cells was carried out by two methods, each of them with very different results: the Lithium Acetate (LiAc) method (Markie, D. (1995). YAC Protocols_[Please complete the reference]; Alison, A. G., D; Kaiser, C; Stearns, T (1997). Methods in Yeast Genetics, Cold Spring Harbor Laboratory Press.) and yeast cell electroporation (Becker, D. M. and L. Guarente (1991). "High-efficiency transformation of yeast by electroporation." Methods Enzymol 194: 182-7)

3.3 Analysis by means of DNA-DNA hybridization (Southern Blot)

The analysis of positive clones was carried out by means of the Southern blot technique. Between 2-4 $\mu$g of genomic DNA were digested with 40-60 U of the corresponding enzyme [*Eco* RV, *Sca* I, *Bgl* II, *Bam* HI, *Hind* III, *Sal* I and *Eco* RI] in 1x digestion buffer with 4 mM spermidine. The digestion was incubated at the enzyme cutting temperature for 12-14 hours. The digestion products were electrophoretically separated in a 0.8% agarose gel in horizontal Horizon cells (20 x 25 cm) (Life Technologies).
The DNA was transferred to a Hybond™-N nylon membrane (Amersham) by means of capillarity. To that end, 14-16 hours in SSC 20X transfer buffer [3 M NaCl, 0.3 M sodium citrate] were needed. The gel was previously treated with a washing in 0.25 N HCl for the depurination of the DNA and two 15-minute washings in 0.4 M for the neutralization and denaturation of the DNA.
Once the DNA was transferred to the membrane, it was fixed by UV irradiation with the CL-1000 *Ultraviolet Crosslinker* (UVP-Stratagen), giving two pulses of 70,000 mJ/cm2.
The single-copy probe was obtained by enzymatic restriction or by means of PCR, and was labeled with radioactivity using 50 ng of DNA, 25 $\mu$Ci of $\alpha$-$^{32}$P-dCTP (Amersham) and the *High Prime* kit (Roche) according to the instructions of the company. To eliminate free nucleotides from the labeled probes, such probes were purified through Probe Quant G50 columns (Amersham). The activity of the probes was quantified in the W 1414 scintillation counter (Beckmann), an activity of approximately 1x10$^6$ cpm/$\mu$l being obtained.
The hybridization was carried out incubating the membrane with the corresponding probe (SEQ ID NO: 68, SEQ ID NO: 69, SEQ ID NO: 72 and SEQ ID NO: 73) for 14-16 hours at 65°C.
The hybridized filters were exposed in exposure cassettes with intensifying screens (Amersham) and photographic films (X-Omat, Kodak) for several days at -80°C. The filters were alternatively exposed in *PhosphorImager* cassettes (Molecular Dynamics) for 1-2 days at room temperature and the signal was analyzed by means of the IQMac v 1.2 program (Molecular Dynamics).

3.4 Small-scale preparation of agarose blocks with yeast cells

To analyze the YAC modification, agarose blocks containing whole cells were prepared, thus achieving that the chromosomes remained intact, and that the size variations experienced by a chromosome can be verified by means of pulsed field electrophoresis. The following protocol was followed:
10 ml of selective medium were inoculated with the yeast clone to be analyzed, allowing it to grow to saturation at 30°C and with stirring (200 r.p.m.). The culture was centrifuged at 3,000 r.p.m. for 5 minutes at room temperature, the supernatant being discarded, and the precipitate was resuspended in 10 ml of 50 mM EDTA pH 8 and centrifuged again at 3,000 r.p.m. for 5 minutes at room temperature. This time, the precipitate was resuspended in 300 $\mu$l of Solution I (1M sorbitol, 20 mM EDTA pH 8, 14 mM $\beta$-mercaptoethanol and 2 mg/ml of zymolyase 20T), attemperated at 40°C. Once attemperated, 50 $\mu$l of a solution of zymolyase 20T at 10 mg/ml were added, then adding 500 $\mu$l of Solution II (Solution I with 2% of agarose, Sea Plaque) also attemperated at 40°C. It was mixed using a pipette with a blue tip cut at the end and it was distributed in the molds using a 200 $\mu$l pipette with a yellow tip which is also cut at the end. 80 $\mu$l per mold were added. They were incubated in ice for 10 minutes so that the agarose solidified and once solidified, the cubes were again transferred to a 10 ml tube with 4 ml of Solution III (1M sorbitol, 20 mM EDTA pH 8, 10 mM Tris pH 7.5, 14 mM $\beta$-mercaptoethanol and 2 mg/ml of zymolyase 20T), incubating at 37°C from 1 to 3 hours, with gentle stirring. Solution III is then changed for 5 ml of Solution IV (1% lauryl sulfate, 100 mM EDTA pH 8, 10 mM Tris pH 8) and it was incubated again for 1 hour at 37°C. Solution IV was subsequently substituted with new solution and incubated at 37°C overnight. The next day the cubes were washed with 5 ml of 20% NDS (0.5 M EDTA, 1 mM Trizma base, 34 mM N-lauryl sarcosine, pH 9) for 2 hours at room temperature, they were changed for new solution and could be stored at 4°C.

3.5 Pulsed field gel electrophoresis (PFGE)

All the gels were prepared at 1% of agarose LMP Sea Plaque in 0.5x TBE buffer (100 mM Tris-Borate, 1 mM EDTA pH 8.3) . The pulsed field electrophoresis system was of Gene Navigator System (Amersham). The yeast markers PFGE marker II (Roche) were used as molecular weight markers, which markers correspond to agarose cubes of *S. cerevisiae* YPH 755 (chromosomes 90, 220, 280, 360, 450, 560, 610, 700, 770, 810, 850, 920, 960, 1,010, 1,100, 1,600 and 2,200 kb).

The initial YAC originally had a size of approximately 650 kb, and the final size after the modifications was 520 kb, therefore a program which optimally separated the chromosomes of this size was used for all the pulsed field electrophoreses which were carried out. The program lasted 26 hours, at 200 V: the first 12 hours with pulses of 50 seconds and the 14 remaining hours with pulses of 100 seconds. Once the gel had finished running, it was stained for 30 minutes in 1-2 liters of electrophoresis buffer with ethidium bromide (EtBr) (0.5 $\mu$g/ml).

For the purpose of analyzing the YAC obtained, the chromosomes were transferred to a HybondTM-N nylon membrane (Amersham) to be analyzed. The transfer was carried out in a manner similar to the transfer already described for Southern blot, except for a few small differences: Prior to the transfer, the gel was subjected to a treatment of 5 minutes to UV light (254 nm) to break the large DNA molecules. Each of them was subsequently incubated twice for 20 minutes with 0.25 N HCL, and then once for 30 minutes with 0.4 M NaOH. The transfer by capillarity was left for at least 48 hours. Both the fixing and the hybridization were carried out as already described in section (3.3).

## 4. Preparation of the YAC for microinjection

### 4.1 Large-scale preparation of agarose blocks with yeast cells

Agarose cubes with a high cell density were prepared in order to isolate a sufficient amount of DNA and generate the transgenic mouse of the invention. To that end, the following process was followed:

200 ml of selective medium were grown with the yeast containing the YAC until saturation. It was centrifuged for 5 minutes at 600 g, eliminating the remaining medium. It was resuspended in 40 ml of 50 mM EDTA (pH 8) per 100 ml of original culture and it was centrifuged again, discarding the supernatant. The yeast precipitate was weighed, assuming a density of 1, the yeasts were taken to a volume of 50% with Solution I and the mixture was attempered at 37°C. Once attempered (about 30 seconds), the same volume of Solution II was added, it was stirred gently and 80 $\mu$l were distributed in the molds, which were being cooled at 4°C. It was allowed to gel for 10 minutes. The cubes were changed to a tube with 8 ml of Solution III per milliliter of cubes and it was incubated for 2 hours at 37°C, with stirring. Solution III was substituted with the same amount of Solution IV and it was incubated for 1 hour at 37°C. It then had to be substituted with new solution and it was left at 37°C overnight. The next day, the cubes were washed at room temperature for 2 hours with 8 ml of 20% NDS per milliliter of cubes. The washing was repeated and they were ready to be loaded.

### 4.2 YAC purification by means of pulsed field

The preparation of the pulsed field for YAC purification required a series of considerations, because the product of this purification was going to be directly used to generate transgenic mice. High sterility working conditions were used, using sterile and disposable material at all times; the region of the gel from which DNA was to be extracted was prevented from coming into contact with EtBr because it has a high mutagenic power, and any solution containing the YAC was pipetted with sterile tips which were cut at the tip to prevent breakages in the DNA molecules.

A large well was prepared by joining seven normal wells of the pulsed field such that 9 cubes can enter. Marker II and ½, ¼, 1/8 and 1/16 cube fractions were loaded into contiguous wells. The gel was run using the program chosen for the separation in this size range (26 hours, at 200 volts; first 12 hours with pulses of 50 s and the remaining hours with pulses of 100 s). Once it was run, the gel was cut into three pieces with a sterile scalpel in the running direction of the gel, thus generating a central piece containing the entire large well, and two side pieces containing the fractions and the markers. These two pieces were stained with EtBr (0.5 $\mu$g/ml), whereas the central piece was kept in running buffer. The stained pieces were used to locate the modified YAC in the fragment which had not been stained. Once the YAC was located, the agarose block containing it was cut and equilibrated in 50 ml of the equilibrium buffer (10 nM Bis-Tris-HCl pH 7.5, 0.1 mM EDTA, 100 mM NaCl, 0.03 mM Spermine and 0.07 mM Spermidine) at 4°C for at least 16 hours. The agarose block containing the modified YAC was then divided into four pieces, which were transferred to 1.5 ml tubes and liquefied by heating for 10 minutes at 70°C, subsequently being attempered at 45°C to add the agarase (GELase™ Epicentre), 5 units per 100 mg of agarose, and it was incubated at 45°C for three hours. The dissolved agarose was then transferred to a Millipore ultracentrifugation column (Ultrafree-®MC Millipore), in which all DNA was concentrated in the column by means of serialized 2-minute centrifugations at 6,000 r.p.m. This operation was repeated until obtaining a final volume of 100 $\mu$l and the column was left overnight at 4°C so that the DNA resuspended. A dialysis filter (Millipore) was subsequently placed in a Petri dish with 40 ml of microinjection buffer (10 mM Tris-HCl pH 7.5, 0.1 mM EDTA pH 8, 100 mM NaCl, 0.03 mM Spermine, 0.07 mM Spermidine and sterile water) and it was allowed to dialyze for 2 or 3 hours. After this time, the drop containing the modified YAC was collected.

**5. Generation of a transgenic mouse by means of intracytoplasmic sperm injection (ICSI), which sperm is incubated with YAC DNA**

The generation of a transgenic mouse comprising the modified YAC was carried out according to the protocols that are extensively described in patent application WO2005/098010 and in Moreira, 2003 (Moreira, P.N., et al., 2003, "Mouse ICSI with frozen-thawed sperm: the impact of sperm freezing procedure and sperm donor strain", Mol Reprod Dev. 66(1): 98-103) and Moreira, 2004 (Moreira, P.N., et al., 2004, "Efficient generation of transgenic mice with yeast artificial chromosomes by intracytoplasmic sperm injection", Biol Reprod. 71(6): 1943-7).

**6. Analysis of founder mice and their descendants**

6.1 Analysis of founder mice by means of PCR

The PCR technique was chosen for carrying out the analysis of the transgene due to the fact that this technique is highly specific and is the quickest for identifying the transgene. Seventeen PCRs of the different exons of the APP gene, two PCRs of the YAC arms and another four PCRs of non-gene encoding regions, were designed for studying the mice. Twenty-three PCRs of all the mice born were carried out according to the protocol described above (1.3) and only those positive for all the PCRs were considered as carrier mice of the entire transgene.

6.2 Transmission to the germ line

The mice considered to be carriers of the transgene were crossed with C57BL/6J mice and their offspring were analyzed. The analysis of the transmission was carried out by means of the PCR of the two YAC arms (LYS2 and TRP1) and of two exons of the APP gene (Exons 6 and 11).

**7. Protein characterization**

7.1 Measurement of the protein concentration

The protein concentration was quantified by the Bradford method using bovine serum albumin (BSA) as a standard.

7.2 Electrophoresis of proteins in polyacrylamide gels in the presence of SDS (SDS-PAGE)

3% concentrating gels and separating polyacrylamide gels with a variable percentage (7.5, 8, 10 or 15%) according to the molecular weight of the proteins to be analyzed were used. 25 mM Tris-HCl pH 6.3, 10% glycerol, 2% SDS, 5% β-mercaptoethanol and 0.01% bromophenol blue was used as sample buffer.
The high molecular weight Rainbow™ colored molecular weight standards from Amershamm Pharmacia (220, 97.4, 66, 46, 30, 21.5 and 14.3 kDa) were used as molecular weight standards.

7.3 Western blot

Briefly, the proteins resolved in polyacrylamide-SDS gels as described in the previous section were transferred to a nitrocellulose filter (Bio-Rad) with a pore size of 0.45 $\mu$m for 90 minutes with a current of 1.3 mA/cm$^2$ by means of the semi-dry electrophoretic transfer method.
After viewing the transferred proteins by means of staining with Ponceau S red, the membrane was saturated overnight at 4°C in PBS with 3% BSA and 0.2% Tween-20 for the purpose of blocking unspecific bindings. The membranes were then incubated for 2 hours at room temperature with the different specific antibodies diluted in PBS with 0.05% Tween-20 (PBST) and 1% BSA. The membranes were washed three times in PBST and incubated for 2 hours at room temperature with secondary antibodies bound to peroxidase diluted 1:50,000 in PBST with 1% BSA. A chemiluminescent method was finally used for the developing, in which peroxidase catalyzes the oxidation of the luminal reagent in the presence of $H_2O_2$ ("ECL" of Amersham).
The bands were quantified by means of densitometry (GS-710 Calibrated Imaging Densitometer, Bio-Rad).

7.4 Antibodies

6E10. 6E10 is a purified mouse monoclonal antibody (Signet) recognizing residues 1-17 of the human Aβ sequence. It has been used for immunolabeling after electrophoresis at a final concentration of 5 $\mu$g/ml.

Anti-$\alpha$-tubulin: Anti-$\alpha$-tubulin (clone B-5-1-2) is a mouse monoclonal antibody (Sigma) recognizing a C-terminal epitope of isoform $\alpha$ of tubulin. It was used for immunolabeling after electrophoresis at a final concentration of 1:10,000.
Biotin-coupled anti-mouse. Purified antibody, generated in horses; it was obtained from Vector Laboratories.

**8. Analysis of the transgene expression in the different tissues**

8.1 Total RNA isolation

The different mice tissue samples were homogenized by means of mechanical rupture and the total RNA was extracted with the commercial "High Pure RNA isolation" kit (Roche diagnostic 1 828 665) according to the protocol indicated by the manufacturer. The assessment of the amount of RNA obtained was carried out in the Nanodrop apparatus (NanoDrop. ND-1000 Spectrophotometer).

8.2 Passing from RNA to DNA by retrotranscription

The retrotranscription was carried out with the "High Capacity cDNA Archive Kit" (Applied Biosystem), for each 1 $\mu$g sample of RNA, 4 units of reverse transcriptase "MultiScribe Reverse Transcriptase", deoxynucleotides (dNTP), a specific buffer were used and random hexamers were used as indicators in the conditions indicated by the manufacturer, in a final volume of 100 $\mu$l. The tubes with the reaction mixture were subjected to the following temperature cycles in the GeneAmp PCR system 2400 or 9600 thermal cycler (Perkin-Elmer):

- Preincubation at 25°C for 10 minutes
- Incubation at 37°C for 120 minutes.

8.3 Quantitative PCR in 384-well plates

Quantitative PCR was carried out for all the prepared tissue samples, which were assayed in triplicate, for which 5 $\mu$l of cDNA, equivalent to 50 ng of original RNA, were used, using the universal conditions described by *Taqman Universal PCR Master Mix, no. AmpErase Ung* (Applied Biosystem). The probes used were: human APP gene, onDemand hAPP Hs00169098_m1 assay; murine APP gene, onDemand mAPP Mn00431827_m1 assay; and the internal control gene (*houseKeeping) gapdh,* onDemand Mn99999915_g1 assay.
The plates with the sample and the reaction mixture were subjected to the temperature cycles specified by the manufacturer, with the ABI PRISM 7900HT Sequence Detection System (Applied Biosystem), which allowed analyzing the samples in real time. The data obtained were analyzed with the SDS 2.1 ABI PRISM program (Applied Biosystem).

**9. Observation under optical microscope: amyloid plaque detection**

Before obtaining samples for histological studies, the mice were subjected to a perfusion and subsequently, the animal was dissected and the brain was extracted.
Condo red staining was carried out with the Accustain amyloid stain kit (Congo Red. Sigma diagnostic), using the protocol recommended by the manufacturer. The amyloid staining can be observed in red or pinkish red, the nuclei in blue and the elastic fibers in lighter red. When the samples were examined with birefringence, they can be observed in green-yellow.

**RESULTS**

The generation of a transgenic mouse endogenously expressing the human APP gene has been developed through the following steps: firstly, carrying out a computer study of YAC b142f9 and its adjacent regions, then eliminating the unknown sequences or the sequences corresponding to other genes present in the YAC, obtaining a modified YAC and subsequently, carrying out a transgenesis to achieve the integration of the entire modified YAC construct, ensuring that it is transmitted to the following generations and finally, verifying the transgene expression in the mice tissues and its levels.

**1. Computer analysis of the APP gene and its environment**

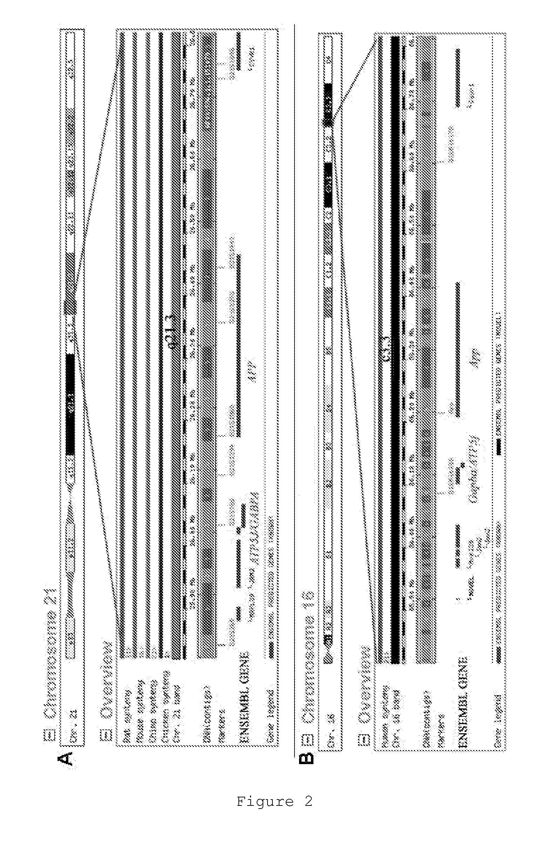

The human APP gene is located in chromosome 21, in the 21q21.3 region, flanked by the CYYR1 gene in 5' and the GABPA and ATP5J genes in 3' (Figure 2). The sequence of this entire region was obtained from the ENSEMBL database (http://www.ensembl.org/), where, in addition to human sequences, the sequences of different organisms, including the *mus musculus* sequence, area available. The hortologous region in *mus musculus* of the APP gene is located in chromosome 16c3.3, flanked by the same genes as in humans (Figure 2), which leads to the supposition that the sequences are quite conserved.

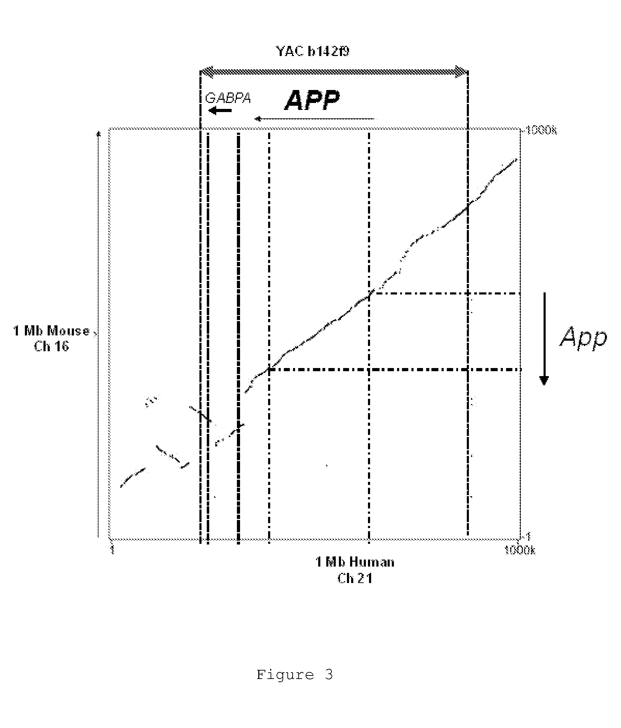

The human APP gene sequence and its environment have been compared with that of the murine gene by means of a homology matrix (Figure 3). Upon comparing 1 Mb of DNA sequence between both species, using the MacVector 7.1 program (Accelrys, Burlington, MA, USA), it is observed that the region encoding the encoding region, like the promoter area of the APP gene, has a high degree of homology, As regards the adjacent regions, in the 5' area of the APP gene less conservation is observed than in the encoding region, but the genome structure is maintained, whereas in the 3' region of the gene, at about 60 kb thereof, there is a series of rearrangements in the murine region in relation to the human region. This particular genome structure is ideal for defining regions which are to be used to modify the YAC, demarcating it in 5' up to a region corresponding to the Seq2 sequence, and in 3' up to the start of the encoding regions of the GABPA gene [Figure 1].

## 2. Choice of the homology sequences for the recombination and cloning in the different vectors

### 2.1 Study of the repeated regions

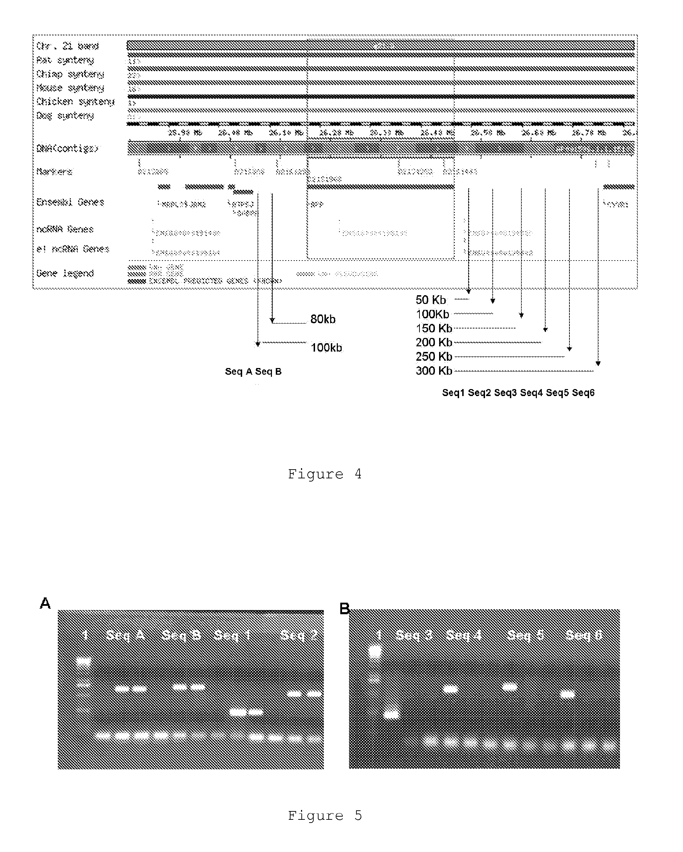

Two conditions are considered to be important when choosing sequences used in the homologous recombination of yeasts: on one hand, the size and on the other hand, its unique condition. As regards the size of the sequence to be chosen, it is normally considered to be suitable to be between 1 and 2 kb. As regards its unique condition, its suitability derives from the fact that the recombination occurs in the desired place. To locate the repetitive sequences and avoid them, the RepeatMasker program (A.F.A. Smith & P. Green, unpublished; http://ftp.genome.washington.edu/cgi-bin/repeatmasker/) was used, analyzing a mega base of the APP gene environment. Regions of 1 to 2 kb, both at 3' and at 5' of the gene were chosen on this sequence, from which repetitive sequences had been eliminated, to be cloned by means of PCR and be used for the homologous recombination in yeasts (Figure 4).

### 2.2 Recombination sequence cloning

The different PCRs of the YAC containing the APP gene (b142f9) were carried out using the oligonucleotides described in section 2 (Figure 4). The YAC extension was verified by means of these PCRs, and was located between sequences 2 and 3, in the 5' region of the APP gene, and sequences A and B located before the GABPA gene in the 3' region of the gene. Sequence 2 was used for the homologous recombination in 5', since this is the sequence in 5' farthest from the APP gene, located in the YAC, which is located at 128,354 kb from the gene transcription initiation site. In the region in 3', both sequence A and B were cloned, since they were located at a distance of 100 and 80 kb, respectively, from the end of the APP gene and were located before the start of the GABPA gene. For the cloning, the PCR of sequences 2, A and B was carried out (Figure 5), the bands corresponding to the selected sequences were purified and were cloned into the PCRII cloning vector (Invitrogen) to obtain pCRII-Seq2, pCRII-SeqA and pCRII-SeqB.

### 2.3 Generation of the recombination vector pB1R-Seq2

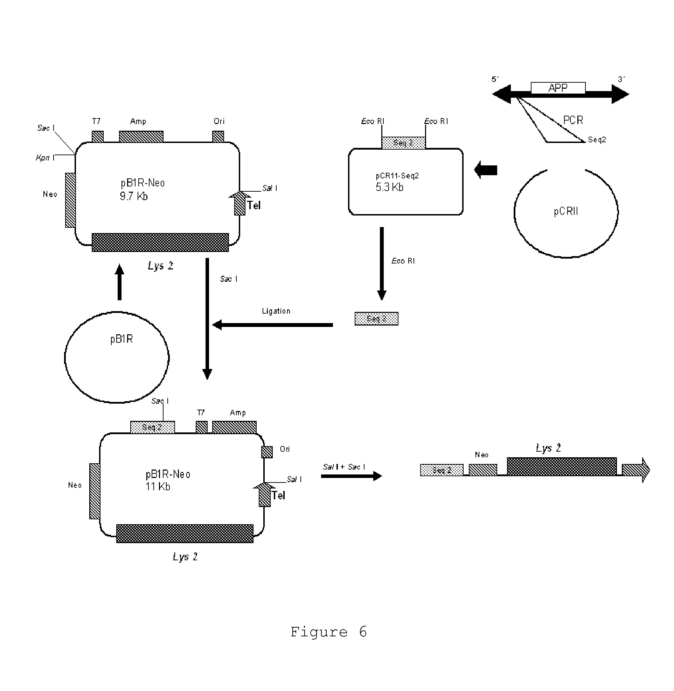

As schematically shown in Figure 6, the plasmid PCRII-Seq2 and a variant of plasmid pB1R were used to generate plasmid pB1R-Seq2 (Lewis, B. C., N. P. Shah, B. S. Braun and C. T. Denny (1992). "Creation of a yeast artificial chromosome fragmentation vector based on lysine-2." Genet Anal Tech Appl 9(3): 86-90). A neomycin selection gene was added to the original plasmid pB1R, such that this YAC, once modified can be used in other types of cultures if necessary. Sequence 2 of the cloning vector, PCRII-Seq2, was extracted by means of digestion with the *EcoRI* enzyme. The ends of this fragment were blunted for their subsequent cloning using the Klenow enzyme. The vector pB1R-Neo was linearized by means of digestion with the *SacI* enzyme. Using the T4 Ligase, the Seq2 fragment was bound to the vector pB1R-Neo, later verifying the correct orientation of the sequence in the vector by means of enzymatic digestion. Said vector was linearized by means of digestion with the *SalI* and *SacI* enzymes, such that it can be used for the 1st homologous recombination in yeasts (Figure 6).

### 2.4. Generation of the recombination vectors pYAC4 3'-SeqA and pYAC4 3'-seqB

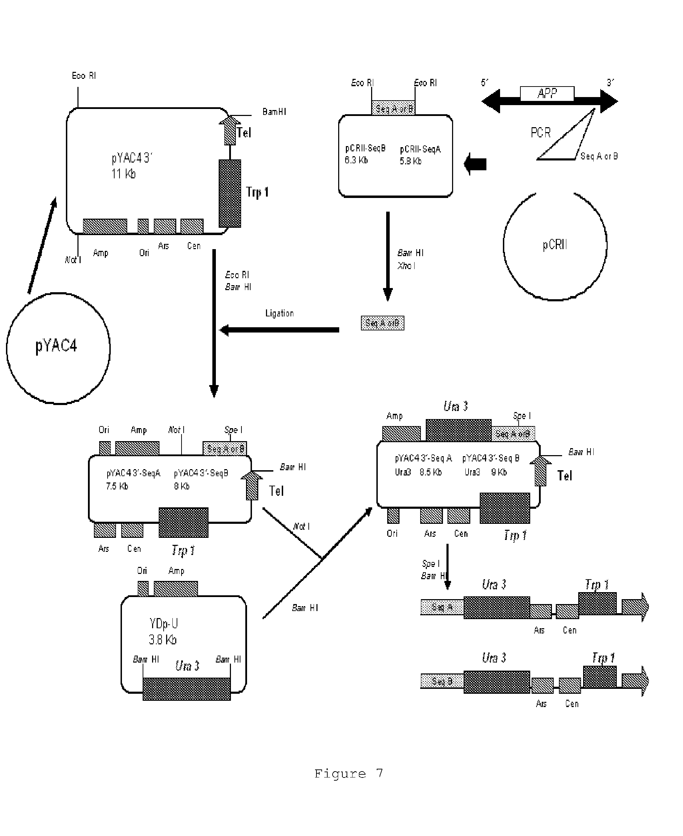

The generation of these two recombination vectors is summarized in Figure 7 (the cloning was carried out in parallel for the two sequences). The recombination sequences were obtained from the plasmids pCRII-SeqA and pCRII-seqB. For the extraction, they were first digested with the XhoI enzyme, blunting the end with the Klenow enzyme and subsequently, they were digested with *BamHI,* such that the sequences had a blunt end and another sticky end. Plasmid pYAC4 3' is a derivative of pYAC4 (Burke, D. T., G. F. Carle and M. V. Olson (1992). "Cloning of large segments of exogenous DNA into yeast by means of artificial chromosome vectors. 1987." Biotechnology 24: 172-8, and Burke, D. T. and M. V. Olson (1991). "Preparation of clone libraries in yeast artificial-chromosome vectors." Methods Enzymol 194: 251-70), generated in the laboratory for studying regulatory sequences of the Tyrosinase gene. By means of digestion, first with *EcoRI*, blunting the ends again and later digesting with *BamHI,* a region which was not of interest was eliminated and the vector was linearized, such that the sequences A and B could be bound in an oriented manner to generate the vectors pYAC4 3'-SeqA and pYAC4 3'-SeqB.

However, the presence of a new auxotrophic marker was necessary so that these vectors could be used in the second homologous recombination. To that end, the URA3 marker of plasmid YDp-U was cloned (Alani, E., L. Cao and N. Kleckner (1987). "A method for gene disruption that allows repeated use of URA3 selection in the construction of multiply disrupted yeast strains." Genetics 116(4): 541-5), first linearizing the vectors pYAC4 3'-Seq and pYAC4 3'-SeqB by means of digesting with *NotI* (again using the Klenow enzyme to blunt the ends) and subsequently, the URA3 gene of plasmid YDp-U was obtained by means digesting with *BamHI,* its ends also being blunted for correct cloning. Using the T4 Ligase, the fragment was cloned into the vector. The linearization of the vectors pYAC4 3'-SeqA URA3 and pYAC4 3'-SeqB URA3 for their correct use in the 2nd recombination of the YAC was carried out by means of enzymatic digestion with *BamHI* and *SpeI* (Figure 7).

### 3. Homologous recombination in the 5' region of the YAC

The chromosomal fragmentation process has been used in this invention to modify the 5' and 3' regions, which consists of eliminating two regions at the ends of the original YAC, one of them being the region containing the GABPA gene in 3'.

#### 3.1 Homologous recombination in YAC b142f9 in the 5' region of the APP gene

A total of 40 colonies was obtained by means of the transformation of the yeasts with LiAc method, using amounts of DNA varying between 100 and 1,000 ng. These 40 colonies were analyzed by means of PCR, obtaining three clones as possible positive clones.

In turn, yeast electroporation resulted in a total of 96 colonies, which were analyzed by means of the PCR technique. None of these showed a correct pattern as a possible positive clone.

#### 3.2 Analysis of the clones resulting from the homologous recombination by means of PCR

The analysis of the clones obtained by means of homologous recombination was carried out by means of designing a hybrid PCR characterized in that one of the two oligonucleotides used is located in the recombination plasmid (in a region which is not located in the YAC) while the other oligonucleotide is located in a region adjacent to the YAC recombination region. The PCR could thus only amplify the fragment when the recombination took place in the expected site. Another vector containing both regions was generated as a positive control. Only three of the 136 analyzed colonies, the colonies numbered 13, 14 and b7, were positive for this PCR.

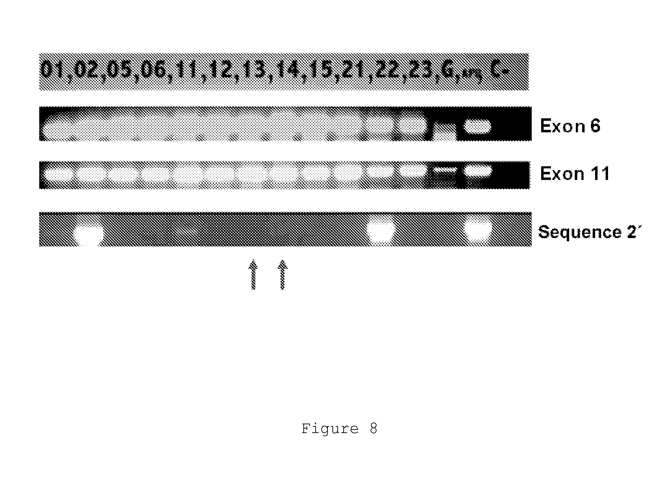

The analysis by means of these clones was completed by means of using another three PCRs, which could provide more information about them. The first two PCRs were carried out to verify if the clones which maintained part of the APP gene were those of the exons 6 and 11 thereof (Figure 8). The third PCR used was that of a sequence located at 5' of the recombination region, which should be negative if the homologous recombination has been correct (Figure 8). The three clones analyzed were positive for the PCRs of exons 6 and 11 and negative for the 5' region, as shown in Figure 8. 3.3 Determination of YAC fragmentation by means of using pulsed field electrophoresis

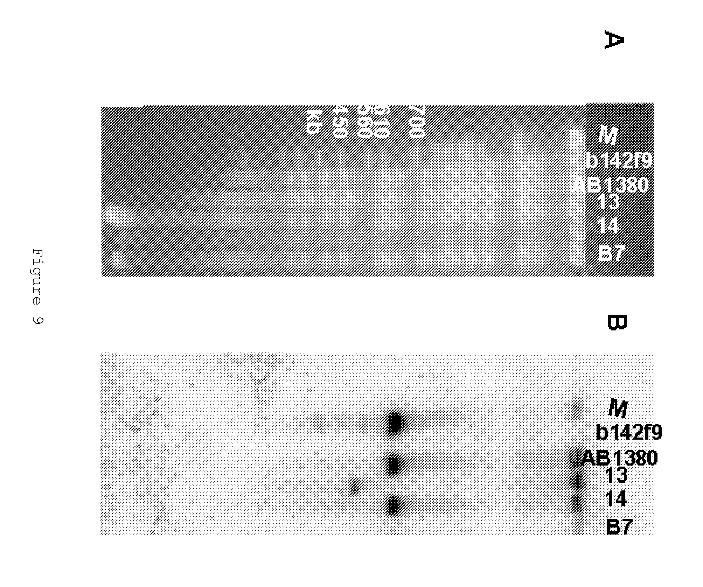

The correct homologous recombination in the YAC must involve a decrease in its size, which can be viewed by checking its electrophoretic mobility. The yeast genome can be separated in a gel, using the pulsed field electrophoresis technique (PFGE). According to this technique, the three analyzed clones were run together with the original clone, a clone of the same yeast strain (which does not contain any YAC) and a molecular weight marker. Figure 9 shows the gel after the run, stained with EtBr. It can be observed between the 680 and 580 kb markers that there is a band in the original YAC clone which is not in the YAC-free yeast clone. This band seems to be maintained in clones 13 and B7, whereas it seems to disappear in clone 14.

The gel was subsequently transferred to a nitrocellulose membrane to carry out a DNA-DNA hybridization with an APP gene cDNA probe, and this verifies the change in electrophoretic mobility with greater certainty. Figure 9 shows how clone 14 has experienced a variation of about 50 kb in the size of the YAC, which variation is the expected one after a correct recombination.

3.4 Analysis of the recombination region by means of the Southern blot technique

YAC fragmentation by means of homologous recombination causes a series of modification in the DNA sequence in the recombination region. The variations between the recombinant clone sequence and the original clone sequence can be analyzed by means of the Southern blot technique. The digestion pattern for *BamHI, ScaI, EcoRV, BglIII* and *HindIII* was different in the two clones.

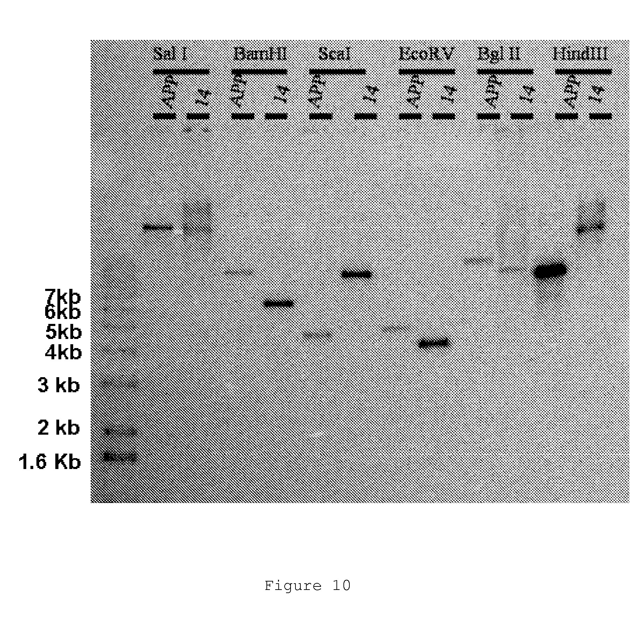

In addition, a variation in the sizes of the bands is obtained in the Southern Blot carried out for the original clone of the APP gene and for clone 14 (Figure 10), which shows that the recombination has occurred in the exact homology site, and together with the results obtained both in the PCR analysis and in the pulsed field electrophoresis, demonstrate a successful recombination in the 5' region of the APP gene and enable using clone 14 as a basis for the next recombination.

3.5 Analysis of the encoding region of clone 14

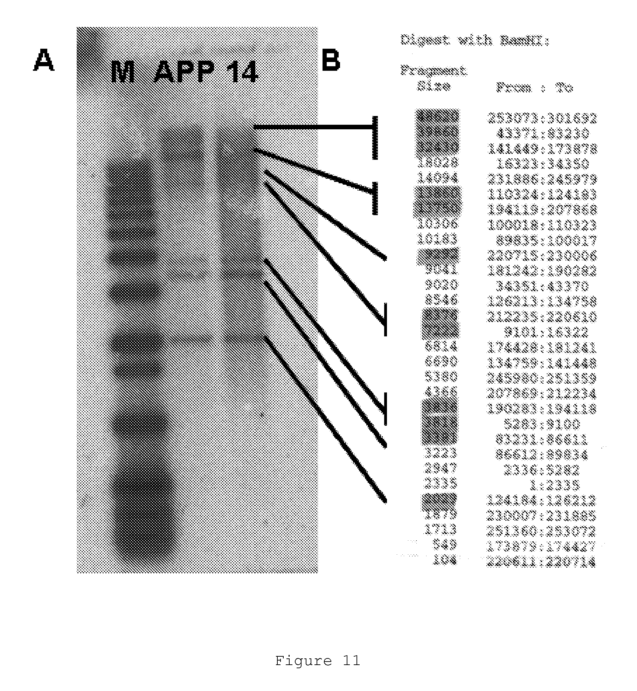

A comparative Southern blot was carried out to ensure that clone 14 had not experienced deletions in the encoding region of the APP gene. For this purpose, the original YAC and the modified YAC were digested with the *BamHI* restriction enzyme, subsequently hybridizing against APP gene cDNA. This hybridization causes a ladder of bands corresponding to the different gene encoding regions and which must be the same for both the original clone and for clone 14. The MacVector program was used to obtain the digestion pattern for the *BamHI* enzyme of the 300 kb of the APP gene. The fragments containing any of the gene encoding regions were then identified. Figure 11 shows the theoretical pattern of this Southern blot and its coincidence with the ladder of bands obtained for the original clone b142f9 and the recombinant clone 14. The conservation of the band pattern obtained for the original clone b142f9 and the recombinant clone 14 indicates that encoding region has not experienced large modification or rearrangements after the recombination.

**4. Homologous recombination in the 3' region of the YAC**

4.1 Homologous recombination in YAC of clone 14. GABPA gene elimination

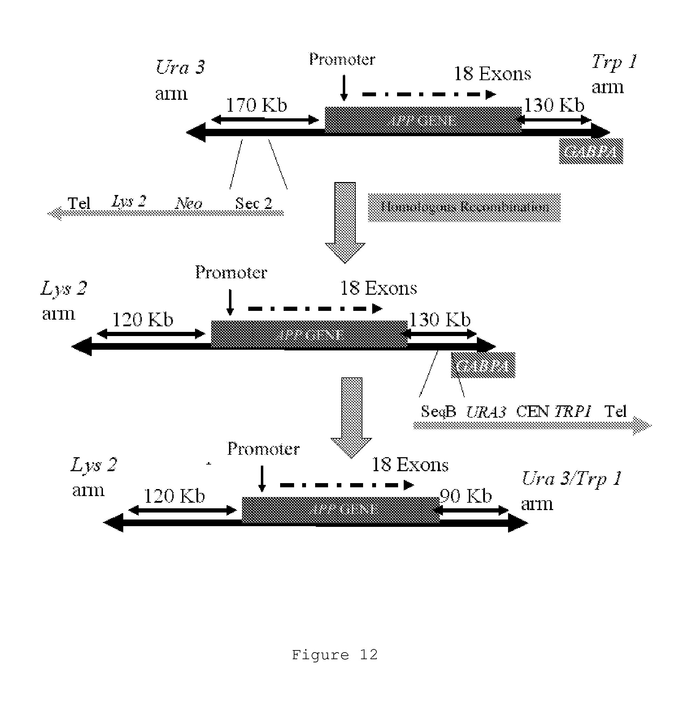

The homologous recombination in the 3' region of the APP gene was carried out on the modified YAC after the first recombination, which required using clone 14 to prepare competent yeasts to be used in the transformation. Two different vectors, pYAC4 3'-SeqA URA3 and pYAC4 3'-SeqB URA3 (Figure 7) were used for this recombination, which vectors only differ in the recombination sequences. Said sequences are separated from one another by 20 kb and are more than 80 kb away from the end of the gene (Figure 4). 40 to 60 kb of the YAC, including the GABPA gene, were eliminated by means of using these recombination sequences (Figure 12).

Given the different efficiency in the transformation of the LiAc and electroporation methods, clearly in favor of the former (see section 3.1 of the previous section), it was decided to be used to carry out the second transformation. Unlike the first recombination, it was necessary to analyze 2,317 colonies for this recombination by means of a dual PCR, a first PCR carried out on exon 6 of the APP gene which should give a positive result, and a second PCR carried out on the GABPA gene which should give a negative result, which would indicate that the sequence corresponding to said gene had been eliminated.

1,720 of the 2,317 colonies analyzed corresponded to sequence A and 597 corresponded to sequence B. After the PCR analysis of the different colonies, 90 of the 1720 and only 5 of the 597 were analyzed by PFGE and Southern blot. The study of these clones by means of PFGE and Southern blot revealed that the homologous recombination carried out with sequence A probably occurred in different regions, which caused strange recombination patterns and which, in several cases, the recombinant YACs had experienced rearrangement processes and fragmentations that were much greater than the expected ones, generating patterns that are very difficult to explain.

The five analyzed clones of the homologous recombination using sequence B provided a clone meeting the necessary requirements to be used in the generation of the transgenic mouse. The different verifications carried out in this clone are described below.

4.2 Analysis by means of the PCR technique, of the clones resulting from the recombination in 3'

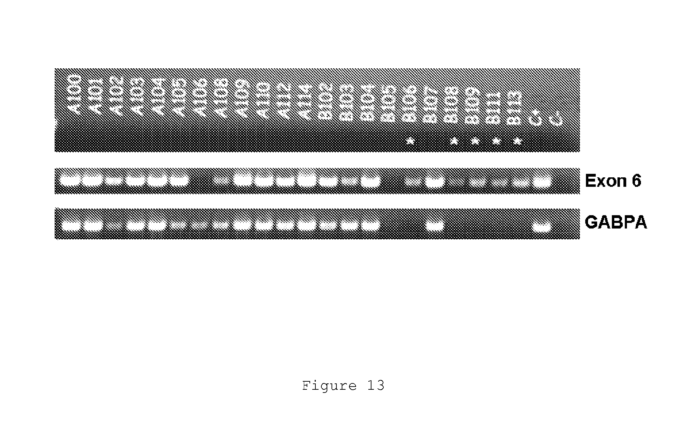

The analysis of the different clones obtained by the yeast transformation was carried out by means of a dual PCR. Figure 13 shows the dual PCR carried out on several clones, both of the recombination with sequence A and the five positives for sequence B. As can be observed, clones b106, b108, b109, b111 and b113 behave as candidates for their subsequent study. The PCR of exon 6 of the APP gene was positive in all of them, which indicates that the clones maintain the YAC with the APP gene and, on the other hand, the PCR of the GABPA gene was negative, which suggests that these clones may have lost the GABPA gene sequences. As will be shown below, only clone b106 of these five clones showed a correct pattern in the homologous recombination.

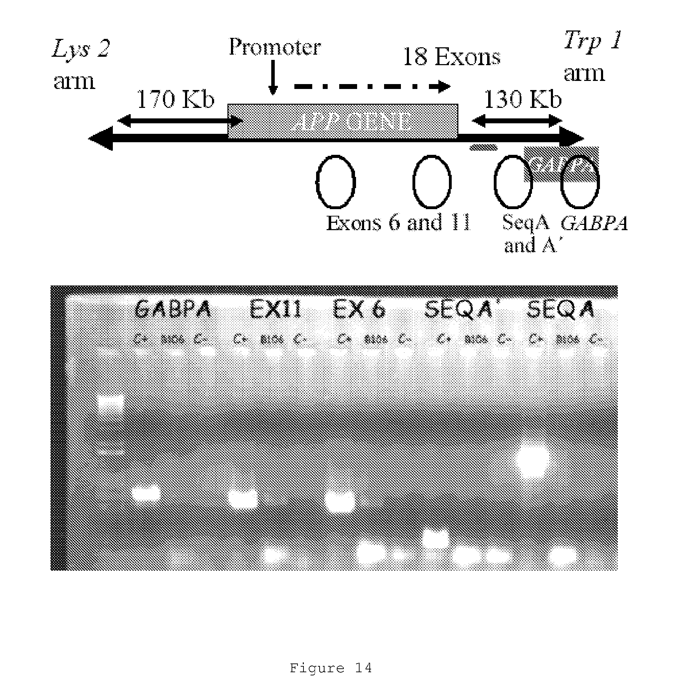

To complete the study of clone b106 by means of PCR, PCRs of two regions located between sequence B and the GABPA gene: sequences A and A', were carried out. In this study, the PCR of the GABPA gene and exon 6 were repeated and that of exon 11 of the APP gene was also included. As can be seen in Figure 14, none of the PCRs in a 3' location with respect to the recombination region was positive, indicating the absence of these sequences in clone b106. The PCRs of the two exons are not as clear as in the case of the positive control, but they have been repeated in several occasions, always obtaining the same positive result.

### 4.3 Determination of the change in electrophoretic mobility of the YAC by means of PFGE

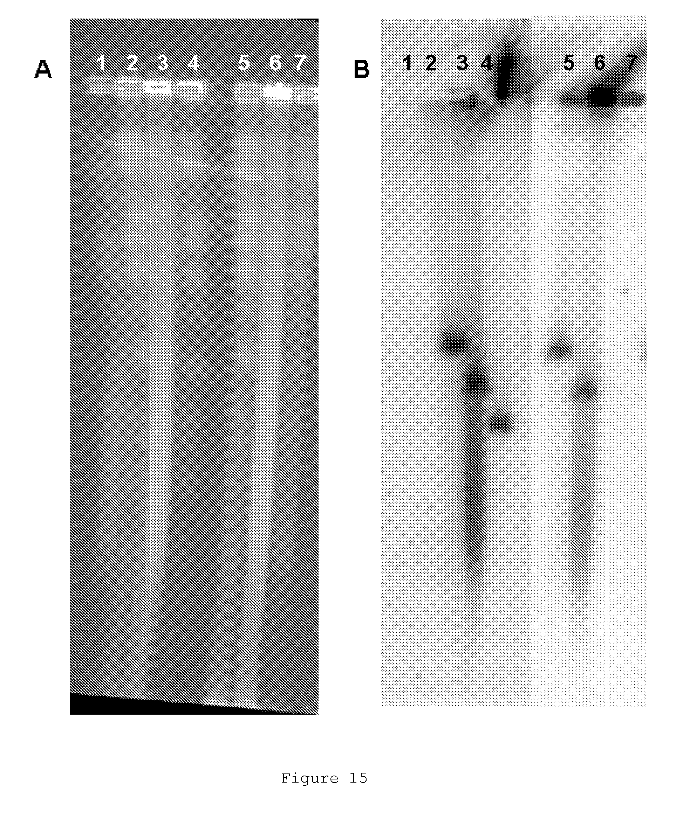

In the same way as after the first homologous recombination, the electrophoretic mobility of the YAC will also change after this second recombination, the resulting YAC having a final size of 520 kb, i.e., 40 kb less than the YAC containing clone 14. Figure 15 shows the 1% agarose gel in which the different clones have been run: the molecular weight marker, the original clone b142f9, clone 14 and clone b106. Said gel was transferred to a nitrocellulose membrane, and was hybridized with APP gene cDNA, achieving viewing the YAC in the different lanes and the variation of the size of the YAC in clone b106, with regard to the original YAC and to clone 14, being observed without any difficulty.

### 4.4 Hybridization of the YAC of clone b106 against GABPA gene cDNA

The hybridization of YAC b106 against GABPA gene cDNA was use to verify that the encoding region of the GABPA gene had been eliminated in said YAC.

Figure 15 shows the pulsed field agarose gel used in the previous section to which other lanes have been added, and the same samples as in the previous lanes have been loaded into these lanes. This time, half of the gel was hybridized with an APP gene cDNA probe and the other half was hybridized with the GABPA gene cDNA probe. As can be seen in said figure, the YAC containing the APP gene of clone b106 does not hybridize for the GABPA gene sequences, whereas the YACs of the original yeast, and that of clone 14 have both genes in the same YAC.

### 4.5 Analysis of the recombination region in 3' by means of the Southern blot technique

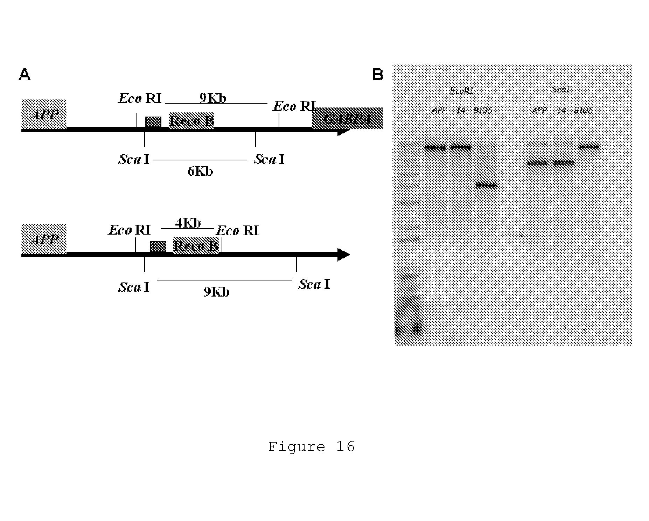

The correct integration of the recombination vector in the YAC was verified by means of the Southern blot technique. This pattern, which is shown in Figure 16, is shared by the original YAC and clone 14, whereas clone b106 has a band pattern that is different but in accordance with the expected diagram. It can be seen in gel hybridization that the band corresponding to YAC b106 has a size different from those of the previous clones, which clearly shows the existing differences in the sequence between the different clones in the region in which the probe hybridizes, which is located in the area around the recombination sequence but at 5' from the latter (Figure 16).

### 4.6 Analysis of the encoding region of clone b106

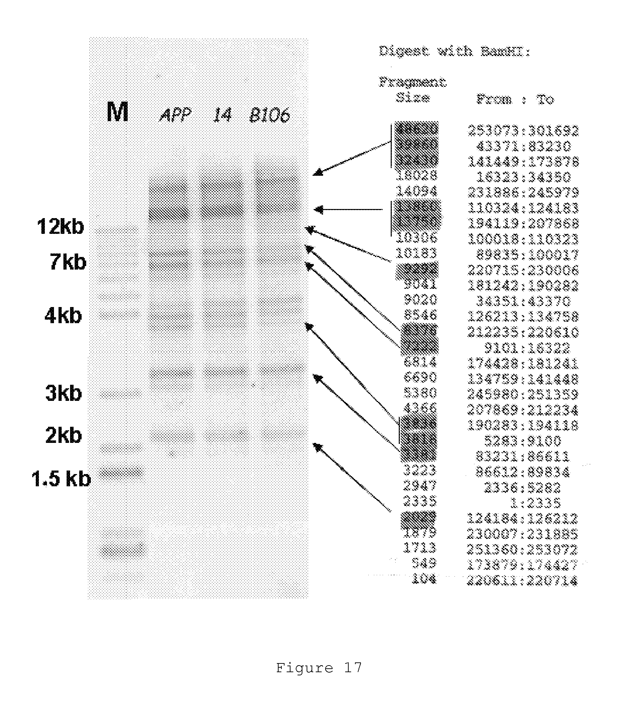

A comparative Southern blot was carried out in order to validate clone b106. If the YAC of clone b106 had only experienced modifications in the 3' region of the YAC but not in the encoding region, the three YACs (the original one, that of clone 14 and that of clone b106) should have the same ladder of bands which was described in Figure 11. If Figure 17, showing the Southern blot carried out, is observed, it can be seen that the band pattern of the three clones is indeed identical and that it is apparently different from that shown in Figure 11. However, these differences are actually simply due to the fact that a better resolution was obtained in the last gel by means of using a cleaner DNA and at a higher concentration. If the band pattern of the gel of Figure 17 is observed, some double and triple bands are better seen.

Nevertheless, the YAC containing the APP gene was what was of interest in clone b106. The same nomenclature used for the clone was used to name this YAC, calling it YAC b106.

### 5. YAC B106 Purification

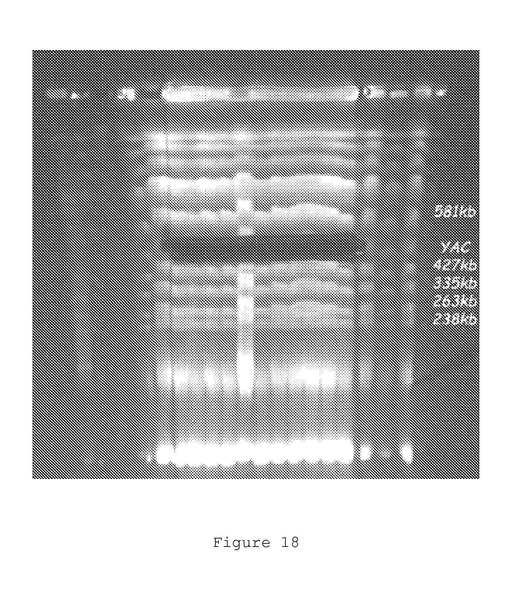

Three consecutive DNA extractions were carried out in this process. As can be seen in Figure 18, showing one of three purifications carried out according to the protocol described in section 4.2 of Material and Methods, the edges of the gel, stained with EtBr, were used to determine the location of YAC b106, and the latter was extracted from the piece of gel which had not been stained. By staining the rest of the gel after the extraction, it could be verified that all the band of YAC b106 had been taken (Figure 18).

The three DNA extractions were measured by means of three different techniques. In the first place, by means of assessment in an agarose gel and the comparison with known amounts of a reference YAC. The results obtained were checked by means of using a fluorimeter and a nanodrop. The concentrations that were obtained for extractions I, II and III were thus 4, 7.1 and 10 ng/μl, respectively.

**6. Transgenesis mediated by intracytoplasmic sperm injection, which sperm is incubated with YAC b106**

Different microinjection sessions were carried out in order to carry out the transgenesis, obtaining a total of 102 animals born after the process had ended. The donor mouse strain of the oocytes used in the transgenesis was a mixed strain, between CD1 and C57BL/6J, whereby greater efficiencies are obtained than by using pure strains. The sperm used comes from the C57BL/6J strain, such that when the founder mice are born, they have a 75% C57BL/6J genetic background.

**7. Analysis of the founder mice**

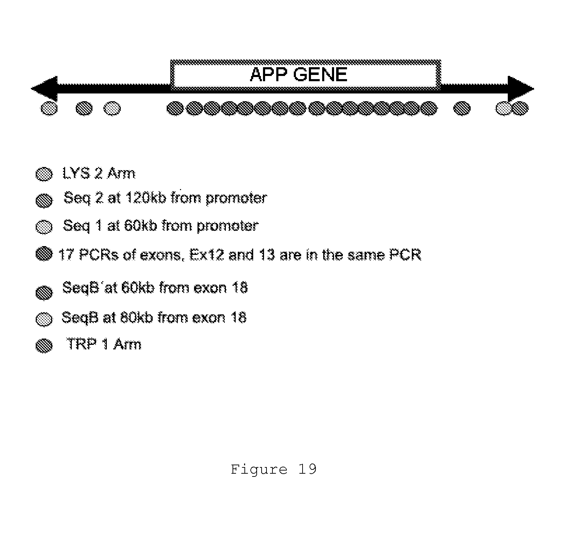

The analysis of the 102 mice obtained after the cytoplasmic injection was carried out using the PCR technique. A scan was carried out over all the YAC regions as a way to reliably verify the insertion of the 520 Kb of the transgene. The following PCRs were designed for this purpose: seventeen PCRs of the exons of the APP gene, one of them containing both exon 12 and 13, two PCRs of the two arms of the YAC, (both for the selection gene of the *LYS2* gene and that of *TRP1*), and another four PCRs of sequences located between the encoding regions and the arms of the YAC (Figure 19).

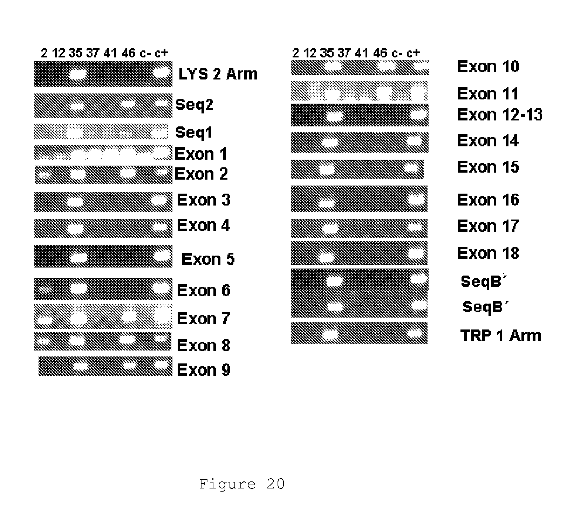

The 23 PCRs of the 102 mice obtained were carried out (Figure 20). A certain degree of transgenesis was obtained in 17 of them, which implies that they have totally or partially integrated the exogenous DNA of the YAC. This DNA integration can be partial, as occurred in fifteen of the cases, but was total in two of them, mouse 35 and female mouse 64, which formed the first transgenic mice obtained in this research with YAC b106, thus complying with one of its main objectives.

**8. Transgene transmission in the First Filial Generation (F1)**

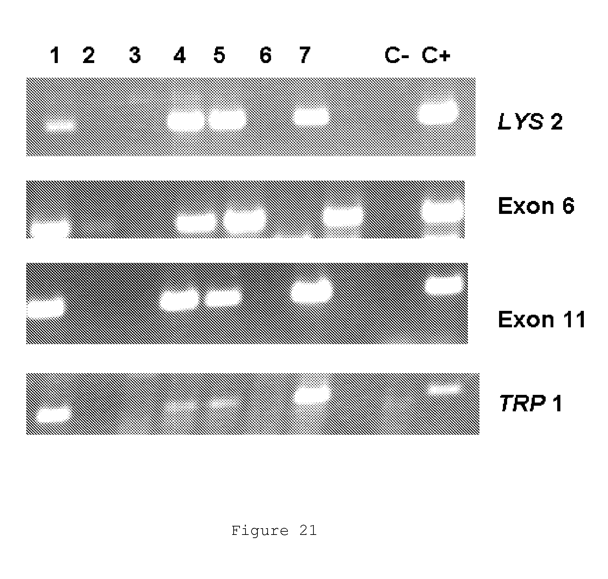

Having checked the generation of two transgenic founder mice, mouse "35" and mouse "64", they were crossed with C57BL/6J mice to check the transgene transmission to their descendants and analyze the expression of such transgene in them. Four PCRs were carried out on the descendants to study YAC b106 transmission: the two PCRs of the arms of the YAC, namely *LYS2* and *TRP1* genes, and the PCRs of exons 6 and 11 of the APP gene. It could thus be verified that the complete YAC was transmitted and that a fractional integration of the YAC had, therefore, not occurred. 41 F1 mice have been analyzed for the descendants of mouse 35 until the drafting of this specification; 18 transgenic mice being identified, which involves a transmission to 44% of the offspring. In the case of female mouse 64, 32 F1 offspring were analyzed, 18 transgenic mice being obtained in her descendants, which involves a 56% transmission. In short, in the two cases, the transgene transmission index to the first generation of descendants of the founders is about 50%, in accordance with those expected according to the Mendelian patterns. Figure 21 shows an illustrative PCR of several offspring of mouse 35.

**9. Conservation of the YAC b106 structure in transgenic mice**

The genomic study of the transgenic mice obtained by crossing the founders was completed by means of verifying the structure of the APP gene encoding region in YAC b106, for which a comparative Southern blot of the descendants of the two founder mice was carried out.

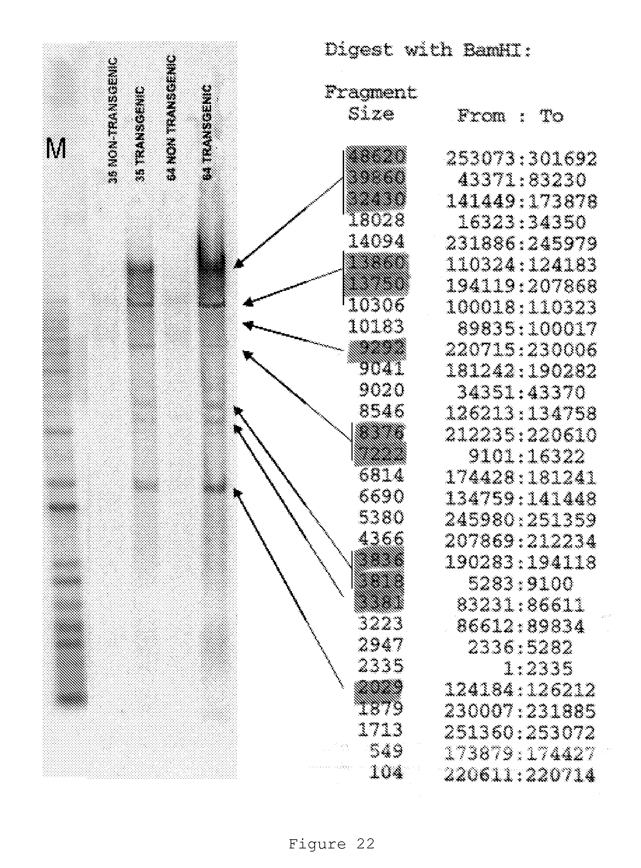

Figure 22 shows the band pattern obtained in F1, or first generation of descendants of founder mice 35 and 64, corresponding to two litter siblings for each founder, one of them being transgenic and the other one being non-transgenic. It can be observed that the pattern is the same as that obtained in Figure 11, which indicates the conservation of the transgene structure in the mouse genome.

**10. Analysis of the human APP gene expression in the transgenic mouse lines**

Once the human APP gene transmission in transgenic lines 35 and 64 has been verified, it was necessary to characterized both RNA and protein expression levels in the different mouse tissues. Mice corresponding to the first generation of line 35 were used for this analysis, using a total of four two-month-old male mice, comparing them with their litter siblings. The study was mainly focused on the central and peripheral nervous system, as these are the tissues in which a greater expression of APP protein occurs. The expression in different mouse body organs was also analyzed, the following being analyzed as a whole: brain, subdivided into four regions which were called cortex, ventricles, cerebellum and mesencephalon (corresponding entirely to their anatomical contents); spinal coed, liver; lung; pancreas;

spleen; small intestine; muscle; testicles; blood; heart and kidney.

10.1. Analysis of the APP gene messenger RNA expression

The first step for verifying the correct human APP gene expression in transgenic line 35 consisted of checking human APP gene transcription in the different mouse tissue by means of RT-PCR. To that end, messenger RNA was extracted from the different mouse tissue and the RNA transcription level was measured by means of real time RT-PCR, using a Taqman probe directed towards the human APP gene. Murine GAPDH gene was used as an internal control of this experiment. To complete the study, the murine APP gene RNA levels were also measured, such that the expression levels of both genes could be compared between the different tissues once they were standardized with GAPDH.

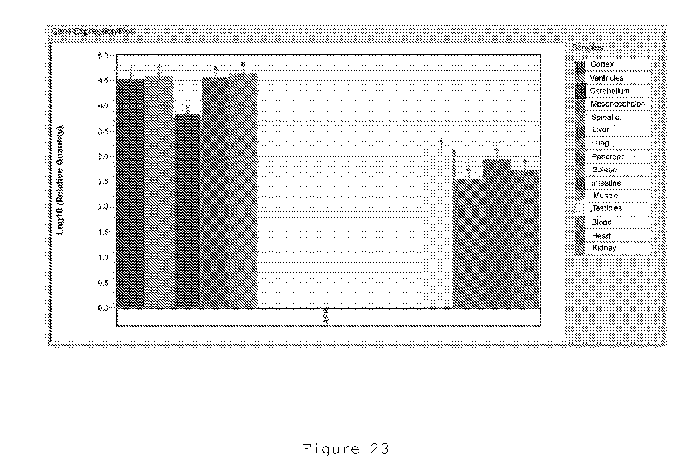

Figure 23 shows the human APP gene messenger RNA levels obtained in the different tissues analyzed. The expression levels are represented in relative logarithmic units, the data having been standardized for the GAPDH gene. It can be observed that this gene is mainly expressed in nervous tissues; the values being very similar in all of them, except in the cerebellum, where they are somewhat reduced. As regards the rest of the studied tissues, detectable expression levels were only reached in testicles, blood, heart and kidney, although in any case they are in an order of magnitude below those detected in the nervous system.

Figure 24 shows the APP gene messenger RNA expression, both of the human and the murine gene, for the transgenic mice and for their litter siblings. Like in the previous graph, the data are standardized with the murine *GAPDH* gene. The results shown indicate a higher gene expression level in the nervous tissue, being reduced in the cerebellum. As regards the rest of the analyzed organs, they show expression in tissues such as the lung, testicles, blood, heart and kidney, whereas in tissues such as the liver, pancreas, intestine, spleen and muscle the levels are much lower and with a very important variation.

10.2 Analysis of the protein level

The human APP protein expression in the mouse means the verification of the correct YAC b106 transcription and translation in the mouse. The protein levels were studied by means of the immunolabeling technique after electrophoresis in polyacrylamide gels (*Western blot*). For this study, an antibody was used which recognized the APP protein but which cannot distinguish between the human and murine form, [Lamb, B. T., et al. (1997). "Altered metabolism of familial Alzheimer's disease-linked amyloid precursor protein variants in yeast artificial chromosome transgenic mice." Hum Mol Genet 6(9): 1535-41.; Lehman, E. J., et al. (2003). "Genetic background regulates beta-amyloid precursor protein processing and beta-amyloid deposition in the mouse." Hum Mol Genet 12(22): 2949-56], therefore the results must be standardized with actin to thus be able to compare the expression levels.

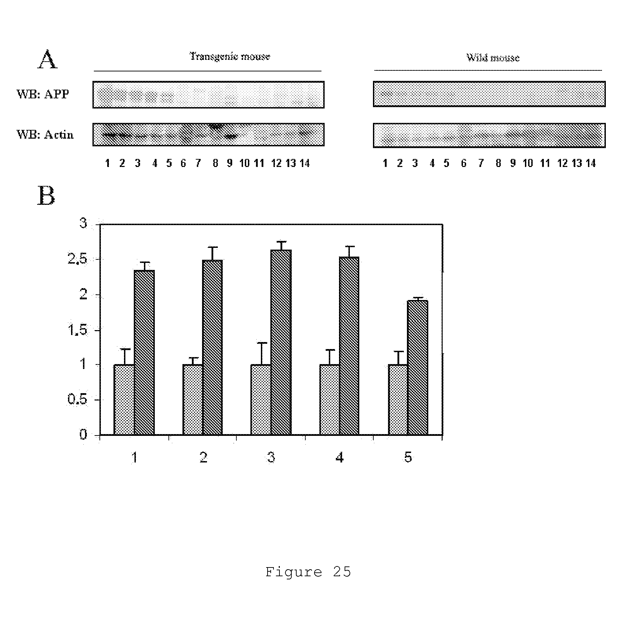

The different tissues of the nervous system have a lack of immunoreactivity, demonstrating the low APP gene expression levels. Figure 25 shows a representative gel for both line 35 and for a wild mouse, the assessment of the expression, standardized with actin, is shown in the bottom graph, in which it is clearly seen that line 35 has at least twice the amount of protein as wild mice. This proportion experiences small variations among the different brain regions analyzed in this study but maintaining those levels.

10.3 Histological study of the mice brains

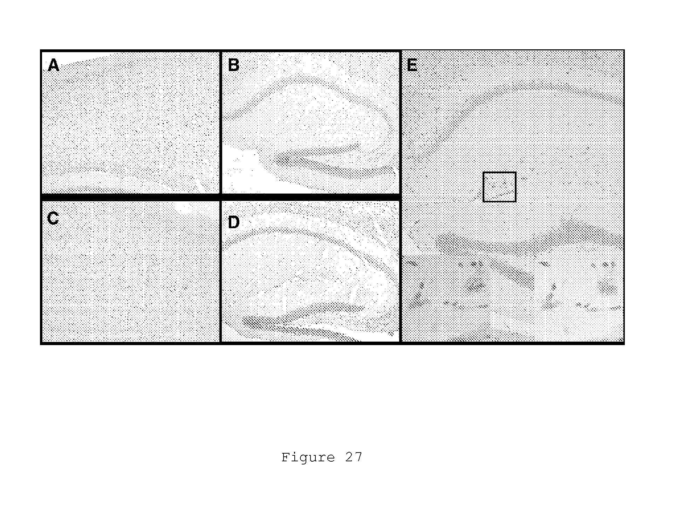

One of the characteristics that the mouse of this invention must have is correct APP protein expression and processing, whereas evidence of an incorrect expression or of an erroneous processing would cause the accumulation of the peptide derived from this protein (Aβ peptide) in the brain. Brain sections of the two mouse lines, with an age of two months, were carried out to complete the study. These sections were stained with Congo red to detect amyloid. Accumulations of any derivative of the APP protein are not detected in any of the two lines or in their litter siblings (Figures 26 and 27), although it is possible to detect them in 1-year-old control transgenic mice (Tg 2576).

**DISCUSSION**

**1. Construction of YAC b106 by means of homologous recombination of yeasts**

Only YAC b142f9, an artificial chromosome with a size of more than 600 kb, which was known to contain the APP gene but it was not known if it also contained other genes which could, as the case may be, introduce perturbations in its subsequent use, was available when designing the transgenic mouse allowing the research of Alzheimer's disease and which has been developed in this research. The publication of the human genome sequence and that of other specific studies carried out on YAC b142f9 allowed proving that the APP gene was not alone in the mentioned YAC, but was accompanied by the GABPA gene. The need thus arises to modify YAC b142f9 in its 5' and 3' regions, eliminating the mentioned GABPA gene but respecting all the APP gene encoding and regulatory regions described to date. This became the first objective of this research in order to be able to use the thus modified YAC in the generation of a transgenic mouse which can be used a model for research on Alzheimer's disease.

The homologous recombination carried out in 5' involved the elimination of 40 kb of the YAC (Figure 12 and Figure 9). Nevertheless, after this elimination, 120 kb were still conserved in the 5' region of the APP gene, which should be enough to allow a correct expression thereof according to the current state of the research. Indeed, although regulatory elements of the APP gene have only been described in the proximal promoter of the gene, the existence of regulatory elements that are farther away is considered to be unquestionable. Therefore, in order to achieve a correct spatial-temporal gene expression, the recombination has been carried out allowing the presence of the evolutionally conserved elements [after analyzing the sequence by means of comparative genomics with that of the mouse gene (Figure 3)]. Sufficient space at the edges has therefor been conserved in the present research in order to assure it.

After demarcating the 5' region by means of homologous recombination in the YAC, the second round of recombination was carried out, in which the GABPA gene was to be directly eliminated by means of chromosomal fragmentation. Its elimination is essential if this YAC is to be used in the generation of an animal model, because the GABPA gene encodes a transcription factor the expression of which in the transgenic mouse could affect the correct development of the mouse in a yet unknown manner which can interfere in the study of the transgenic phenotype, making it impossible to assure which gene is responsible for said phenotype.

The homologous recombination in the 3' region of the APP gene was much more complicated than that carried out in 5', possibly due to the presence of secondary DNA structures in the 3' region which have made the access to the recombination sequences more complicated.

As in the case of the first recombination, a series of experiments was designed to reliably verify the correct homologous recombination and the elimination of the GABPA gene. The first colony screening was carried out by means of a dual PCR. This method allowed discriminating between the hundreds of colonies obtained and reducing this number by one order of magnitude. Although apparently the colonies that did not have the GABPA gene but did have exon 6 should be considered suitable, subsequent analyses by means of PFGE showed that was not always true.

In this sense, the analysis by means of PFGE and the subsequent hybridization of the filters with the APP gene cDNA and the GABPA gene cDNA was the most reliable and conclusive analysis of them all. This is because, indeed, the change in the electrophoretic mobility of YAC b106 could only be due to a fragmentation of the YAC of clone 14. The hybridization with the GABPA gene cDNA further allow recognizing all the gene encoding regions, whereas the PCR was only directed to one of the exons. The verification by means of hybridization of the disappearance of the sequences encoding the GABPA gene of the YAC b106 entailed, together with the expected change of electrophoretic mobility, the confirmation that this colony had experienced the planned recombination. Nevertheless, the verification by means of *Southern blot* and different PCRs were useful for completing the study and thus passing to the process for generating the transgenic mouse.

## 2. Generation of transgenic mice by means of ICSI

Transgenic mice with YAC b106 of 520 kb were obtained by means of ICSI according to processes described in patent application WO2005/098010 and in Moreira, 2003 and 2004 (mentioned above). A total of 17 transgenic mice, i.e., carriers of any DNA fragment of YAC b106Of, were obtained out of the 102 mice born and analyzed.

The verification of the complete integration of the 520 kb construct required a careful analysis throughout the entire sequence, for which 23 PCRs were carried out on each mouse throughout the entire YAC. The group of PCRs designed to verify the complete integration of the YAC (Figure 19) was only positive for mice 35 and 64, which identified these mice as the possible founders of the new animal model.

However, the presence of the transgene in the founder mice does not ensure its presence in the germ line, which condition is essential for its subsequent use, therefore its transmission must be verified in the following generations. To check it, the clones were crossed with C57BL/6J mice and all the litters produced by the two transgenic lines(Figure 21) were analyzed by means of PCR, a transmission of approximately 50% in the first generation or F1 being obtained (section 8 of the Results). The verification of the transgene transmission in the germ line (Figure 21) as well as the expression of such transgene (Figure 23) demonstrated that the group of PCRs designed to verify the integration of the YAC had been a suitable strategy.

## 3. Human APP gene expression in the mouse

The first step to characterize the human APP gene expression in the mouse was carried out by means of analyzing the gene RNA expression. To that end, a quantitative plate PCR technique was used to compare the levels between the different tissues. According to the existing references, the expression of this gene occurs in several tissues but these levels are higher in the brain. And, indeed, as can be seen in Figure 23, line 35 expression levels shows the highest values in the brain in the different regions which have been analyzed. The high gene expression index, registered in the spinal cord, must be emphasized, and this data should not be surprising because it is formed by nervous tissues where the cell lines producing this protein are located. Therefore, the results of this research as regards the human APP gene expression in mice are fully consistent with those described previously for similar mice, in which the highest RNA expression levels are in the brain.

The human APP gene expression in the different organs is to a great extent similar to the murine APP gene expression (Figure 24), conserving the expression pattern in the nervous tissues, as well as in the heart, blood, testicles and kidney. However, the murine APP gene expression is detected in the liver, lung, pancreas, intestine and muscle, although with values that are much lower than the other organs (one order or magnitude lower), except in the case of the lung, where there is an expression similar to that of the heart, blood, testicles and kidney, which did not occur with the human APP gene, the expression of which was not detected in those organs, as has already been pointed out.

Once the gene expression in the different mouse tissues has been verified, the translation of said RNA to protein had to be confirmed. A Western Blot analysis of the amount of protein present in the different tissues which had been analyzed by means of RT-PCR was carried out for this study. The study was initially dealt with by using the 6E10 antibody, specific for the human protein, but said specificity could not be reproduced in the experiments of this research, therefore the decision was made to use the 22C11 antibody, which recognizes both the human and the murine proteins, and thus the total amount of protein could be assessed.

The high protein expression in the nervous tissues made the identification and assessment of such protein possible, whereas in the other tissues, with much lower registers of protein levels, its identification was impossible (Figure 25). After the assessment of the protein levels in the nervous tissues and their standardization with actin values, the transgenic mice and their non-transgenic siblings protein level were compared, observing that in the former, values were found that were between two and two and a half times higher with regard to those of the endogenous protein, depending on the specific tissue. Thus, in the cortex, ventricles and spinal cord, the protein levels were slightly above two times with regard to the endogenous protein, whereas in the mesencephalon and cerebellum, the proportion increased, reaching two and a half times. It must be considered that both the human and the murine protein are being detected in the transgenic mice, therefore a total APP protein expression of two times means that the same amount of human and murine protein is being expressed. These expression differences between the two genes is slightly higher than previous descriptions, which register expression levels between 1.7 and 2 times that of the endogenous gene, measured only in the cortex region, which difference can be due to the mouse strain used, the genetics of which can affect both the levels and the processing thereof.

Finally, a preliminary study was carried out on the mice brains, attempting to detect some of the typical neuropathologies of the disease. For the time being, no sign of amyloid peptide deposits has been detected in these preliminary studies in two-month-old mice, which is consistent with the previous studies in mice with these characteristics.

**SEQUENCE LISTING**

<110>  UNIVERSIDAD AUTÓNOMA DE MADRID

<110>  CONSEJO SUPERIOR DE INVESTIGACIONES CIENTÍFICAS


<120> ALZHEIMER'S DISEASE ANIMAL MODEL, METHOD FOR OBTAINING
      SAME AND USES THEREOF


<160>  73


<170>  PatentIn version 3.1


<210> 1
<211> 25
<212> DNA
<213> Artificial sequence


<223> Oligonucleotide APP3´-B-U used to delimit the 5' and 3'
regions flanking the APP gene in the YAC.


<400> 1
atcctaagag gaagggatct taagg                                    25



<210> 2
<211> 25
<212> DNA
<213> Artificial sequence


<223> Oligonucleotide APP3´-B-L used to delimit the 5' and 3'
regions flanking the APP gene in the YAC.


<400> 2
gggagaacaa acatacctca ctagc                                    25

<210> 3
<211> 24
<212> DNA
<213> Artificial sequence

<223> Oligonucleotide APP3´-B-U used to delimit the 5' and 3' regions flanking the APP gene in the YAC.

<400> 3
ccagcatgtt ttccaaagta tgag                              24


<210> 4
<211> 25
<212> DNA
<213> Artificial sequence

<223> Oligonucleotide APP3´-B-L used to delimit the 5' and 3' regions flanking the APP gene in the YAC.

<400> 4
tgaaatgtcc ccacactgat cactg                             25

<210> 5
<211> 30
<212> DNA
<213> Artificial sequence

<223> Oligonucleotide APP3´-1-U used to delimit the 5' and 3' regions flanking the APP gene in the YAC.

<400> 5
gtatagtatg gatggttcaa acagagccta                        30

```
<210> 6
<211> 30
<212> DNA
<213> Artificial sequence


<223> Oligonucleotide APP3´-1-L used to delimit the 5' and 3'
regions flanking the APP gene in the YAC.


<400> 6
aaggtaaagc tctagaatct atcagtgcct                       30



<210> 7
<211> 30
<212> DNA
<213> Artificial sequence


<223> Oligonucleotide APP3´-2-U used to delimit the 5' and 3'
regions flanking the APP gene in the YAC.


<400> 7
tttagaagag ctcagtaaga atccacattt                       30



<210> 8
<211> 30
<212> DNA
<213> Artificial sequence


<223> Oligonucleotide APP3´-2-L used to delimit the 5' and 3'
regions flanking the APP gene in the YAC.


<400> 8
cattttgtcg ttactagtac cattggtttc                       30
```

```
<210> 9
<211> 25
<212> DNA
<213> Artificial sequence


<223> Oligonucleotide APP5´-1-U used to delimit the 5' and 3'
regions flanking the APP gene in the YAC.


<400> 9
ctcaaaaaca gcaacacaga tgtgc                                    25



<210> 10
<211> 22
<212> DNA
<213> Artificial sequence


<223> Oligonucleotide APP5´-1-L used to delimit the 5' and 3'
regions flanking the APP gene in the YAC.


<400> 10
gagcttaaac accttcctct gc                                     22



<210> 11
<211> 25
<212> DNA
<213> Artificial sequence


<223> Oligonucleotide APP5´-2-U used to delimit the 5' and 3'
regions flanking the APP gene in the YAC.


<400> 11
tggggagagg aatggaattt ggagc                                    25
```

<210> 12

<211> 25

<212> DNA

<213> Artificial sequence


<223> Oligonucleotide APP5´-2-L used to delimit the 5' and 3' regions flanking the APP gene in the YAC.


<400> 12

gtcatttcca gaaaaagccc actgc                                             25


<210> 13

<211> 25

<212> DNA

<213> Artificial sequence


<223> Oligonucleotide APP5´-3-U used to delimit the 5' and 3' regions flanking the APP gene in the YAC.


<400> 13

tcaaggacat ggagattggg caggc                                             25



<210> 14

<211> 25

<212> DNA

<213> Artificial sequence


<223> Oligonucleotide APP5´-3-L used to delimit the 5' and 3' regions flanking the APP gene in the YAC.


<400> 14

ggggtaacag gttgccggtc atatg                                             25

<210> 15
<211> 25
<212> DNA
<213> Artificial sequence

<223> Oligonucleotide APP5´-4-U used to delimit the 5' and 3' regions flanking the APP gene in the YAC.

<400> 15
caccagacca tgatgtctca gtagc                                    25

<210> 16
<211> 25
<212> DNA
<213> Artificial sequence

<223> Oligonucleotide APP5´-4-L used to delimit the 5' and 3' regions flanking the APP gene in the YAC.

<400> 16
ctgcaaaaca gccctcatat tctgc                                    25

<210> 17
<211> 25
<212> DNA
<213> Artificial sequence

<223> Oligonucleotide APP5´-5-U used to delimit the 5' and 3' regions flanking the APP gene in the YAC.

<400> 17
tagcatcctt tgctaagcca gttgc                                    25

```
<210> 18
<211> 25
<212> DNA
<213> Artificial sequence

<223> Oligonucleotide APP5´-5-L used to delimit the 5' and 3'
regions flanking the APP gene in the YAC.

<400> 18
gcttgttatt ggaggttcca gcacg                                    25


<210> 19
<211> 25
<212> DNA
<213> Artificial sequence

<223> Oligonucleotide APP5´-6-U used to delimit the 5' and 3'
regions flanking the APP gene in the YAC.

<400> 19
tccaacgggg gagtgagtga aaggc                                    25



<210> 20
<211> 25
<212> DNA
<213> Artificial sequence

<223> Oligonucleotide APP5´-6-L used to delimit the 5' and 3'
regions flanking the APP gene in the YAC.

<400> 20
gcctcactcc tgcaaacgtg cccaa                                    25
```

```
<210> 21
<211> 25
<212> DNA
<213> Artificial sequence


<223> Oligonucleotide APP5´-7-U used to delimit the 5' and 3'
regions flanking the APP gene in the YAC.


<400> 21
tctttcttcc accttggtat cctgc                                    25



<210> 22
<211> 25
<212> DNA
<213> Artificial sequence


<223> Oligonucleotide APP5´-7-L used to delimit the 5' and 3'
regions flanking the APP gene in the YAC.


<400> 22
caggatgtct ctggattttt actcg                                    25



<210> 23
<211> 21
<212> DNA
<213> Artificial sequence


<223> Oligonucleotide APP Ex1-U used to check the exons of the
APP gene, as well as the presence of the GABPA gene.


<400> 23
agtttcctcg gcagcggtag g                                        21
```

```
<210> 24
<211> 21
<212> DNA
<213> Artificial sequence

<223> Oligonucleotide APP Ex1-L used to check the exons of the
APP gene, as well as the presence of the GABPA gene.

<400> 24
ccagcaggag cagtgccaaa c                                          21


<210> 25
<211> 20
<212> DNA
<213> Artificial sequence

<223> Oligonucleotide APP-EX2-U used to delimit the 5' and 3'
regions flanking the APP gene in the YAC.

<400> 25
aagaccgggc tgattcctaa                                            20


<210> 26
<211> 20
<212> DNA
<213> Artificial sequence

<223> Oligonucleotide APP-EX2-L used to delimit the 5' and 3'
regions flanking the APP gene in the YAC.

<400> 26
```

```
tccaacgtga attgctagcc                                        20
```

<210> 27

<211> 20

<212> DNA

<213> Artificial sequence

<223> Oligonucleotide APP-EX3-U used to delimit the 5' and 3' regions flanking the APP gene in the YAC.

<400> 27

```
cccaagcatt ttggataagg                                        20
```

<210> 28

<211> 20

<212> DNA

<213> Artificial sequence

<223> Oligonucleotide APP-EX3-L used to delimit the 5' and 3' regions flanking the APP gene in the YAC.

<400> 28

```
cctctttttc ttccctcaag                                        20
```

<210> 29

<211> 20

<212> DNA

<213> Artificial sequence

<223> Oligonucleotide APP-EX4-U used to delimit the 5' and 3' regions flanking the APP gene in the YAC.

<400> 29

```
ttgattgggt tgcttaggca                                              20
```

```
<210> 30
<211> 20
<212> DNA
<213> Artificial sequence
```

```
<223> Oligonucleotide APP-EX4-L used to delimit the 5' and 3'
regions flanking the APP gene in the YAC.
```

```
<400> 30
tgttgcctca aaatacccct                                              20
```

```
<210> 31
<211> 20
<212> DNA
<213> Artificial sequence
```

```
<223> Oligonucleotide APP-EX5-U used to delimit the 5' and 3'
regions flanking the APP gene in the YAC.
```

```
<400> 31
ctaccactca ctgttttctc                                              20
```

```
<210> 32
<211> 20
<212> DNA
<213> Artificial sequence
```

```
<223> Oligonucleotide APP-EX5-L used to delimit the 5' and 3'
regions flanking the APP gene in the YAC.
```

```
<400> 32
gcagagacct tttcagtgat                                        20


<210> 33
<211> 21
<212> DNA
<213> Artificial sequence


<223> Oligonucleotide APP-EX6-U used to delimit the 5' and 3'
regions flanking the APP gene in the YAC.


<400> 33
tgccaaaatt ccatatggac g                                      21


<210> 34
<211> 21
<212> DNA
<213> Artificial sequence


<223> Oligonucleotide APP-EX6-L used to delimit the 5' and 3'
regions flanking the APP gene in the YAC.


<400> 34
gggatttgcc aagcagcata t                                      21


<210> 35
<211> 20
<212> DNA
<213> Artificial sequence


<223> Oligonucleotide APP-EX7-U used to delimit the 5' and 3'
regions flanking the APP gene in the YAC.
```

```
<400> 35
ccactgggag gattaaaaga                                        20


<210> 36
<211> 20
<212> DNA
<213> Artificial sequence


<223> Oligonucleotide APP-EX7-L used to delimit the 5' and 3'
regions flanking the APP gene in the YAC.


<400> 36
gagaagtgga cagaaatgtg                                        20


<210> 37
<211> 20
<212> DNA
<213> Artificial sequence


<223> Oligonucleotide APP-EX8-U used to delimit the 5' and 3'
regions flanking the APP gene in the YAC.


<400> 37
tgtcagtgga ctcgtgcatt                                        20


<210> 38
<211> 20
<212> DNA
<213> Artificial sequence


<223> Oligonucleotide APP-EX8-L used to delimit the 5' and 3'
```

regions flanking the APP gene in the YAC.

<400> 38
catctcaagc tgtctggcaa                                    20


<210> 39
<211> 20
<212> DNA
<213> Artificial sequence


<223> Oligonucleotide APP-EX9-U used to delimit the 5' and 3' regions flanking the APP gene in the YAC.


<400> 39
catgtcttca gcaccaactg                                    20


<210> 40
<211> 20
<212> DNA
<213> Artificial sequence


<223> Oligonucleotide APP-EX9-L used to delimit the 5' and 3' regions flanking the APP gene in the YAC.


<400> 40
caaactgtgc ccacacagta                                    20


<210> 41
<211> 20
<212> DNA
<213> Artificial sequence

<223> Oligonucleotide APP-Ex10-U used to delimit the 5' and 3' regions flanking the APP gene in the YAC.

<400> 41
cagataggaa ggggtatgta                                        20

<210> 42
<211> 20
<212> DNA
<213> Artificial sequence

<223> Oligonucleotide APP-Ex10-L used to delimit the 5' and 3' regions flanking the APP gene in the YAC.

<400> 42
ggagcaaata taaggcagga                                        20

<210> 43
<211> 21
<212> DNA
<213> Artificial sequence

<223> Oligonucleotide APP-EX11-U used to delimit the 5' and 3' regions flanking the APP gene in the YAC.

<400> 43
tgatgagggt tggagagtgc a                                      21

<210> 44
<211> 21
<212> DNA
<213> Artificial sequence

<223> Oligonucleotide APP-EX11-L used to delimit the 5' and 3' regions flanking the APP gene in the YAC.

<400> 44

caagatggaa tggacagggg t                                      21

<210> 45
<211> 20
<212> DNA
<213> Artificial sequence

<223> Oligonucleotide APPEx12-13-U used to delimit the 5' and 3' regions flanking the APP gene in the YAC.

<400> 45

cctcgtcacg tgttcaatat                                        20

<210> 46
<211> 20
<212> DNA
<213> Artificial sequence

<223> Oligonucleotide APPEx12-13-L used to delimit the 5' and 3' regions flanking the APP gene in the YAC.

<400> 46

caacttcatc ctgaatctcc                                        20

<210> 47
<211> 20
<212> DNA

<213> Artificial sequence

<223> Oligonucleotide APPEx14-U used to delimit the 5' and 3' regions flanking the APP gene in the YAC.

<400> 47
cacgtgaaag cagttgaagt                               20

<210> 48
<211> 20
<212> DNA
<213> Artificial sequence

<223> Oligonucleotide APPEx14-L used to delimit the 5' and 3' regions flanking the APP gene in the YAC.

<400> 48
ttgccaccta tacaatggag                               20

<210> 49
<211> 20
<212> DNA
<213> Artificial sequence

<223> Oligonucleotide APPEx15-U used to delimit the 5' and 3' regions flanking the APP gene in the YAC.

<400> 49
ctggcacatc aatagcgata                               20

<210> 50
<211> 20
<212> DNA

<213> Artificial sequence

<223> Oligonucleotide APPEx15-L used to delimit the 5' and 3' regions flanking the APP gene in the YAC.

<400> 50
actcggaact tgggaaatga                                          20

<210> 51
<211> 20
<212> DNA
<213> Artificial sequence

<223> Oligonucleotide APPEx16-U used to delimit the 5' and 3' regions flanking the APP gene in the YAC.

<400> 51
taaaggcagc agaagcctta                                          20

<210> 52
<211> 20
<212> DNA
<213> Artificial sequence

<223> Oligonucleotide APPEx16-L used to delimit the 5' and 3' regions flanking the APP gene in the YAC.

<400> 52
gctcagccta gcctatttat                                          20

<210> 53
<211> 20

```
<212> DNA
<213> Artificial sequence

<223> Oligonucleotide APPEx17-U used to delimit the 5' and 3'
regions flanking the APP gene in the YAC.

<400> 53
accagttggg cagagaatat                                        20


<210> 54
<211> 20
<212> DNA
<213> Artificial sequence

<223> Oligonucleotide APPEx17-L used to delimit the 5' and 3'
regions flanking the APP gene in the YAC.

<400> 54
cttgagcaga atattcacgg                                        20


<210> 55
<211> 20
<212> DNA
<213> Artificial sequence

<223> Oligonucleotide APPEx18-U used to delimit the 5' and 3'
regions flanking the APP gene in the YAC.

<400> 55
cttggtaatt gaagaccagc                                        20


<210> 56
```

```
<211> 20
<212> DNA
<213> Artificial sequence

<223> Oligonucleotide APPEx18-L used to delimit the 5' and 3'
regions flanking the APP gene in the YAC.

<400> 56
cttttgacag ctgtgctgta                                          20



<210> 57
<211> 20
<212> DNA
<213> Artificial sequence

<223> Oligonucleotide GABPA-EX-U used to delimit the 5' and 3'
regions flanking the APP gene in the YAC.

<400> 57
tattattacg atggggacat                                          20



<210> 58
<211> 20
<212> DNA
<213> Artificial sequence

<223> Oligonucleotide GABPA-EX-L used to delimit the 5' and 3'
regions flanking the APP gene in the YAC.

<400> 58
gtaaagcaac atctaaagca                                          20
```

```
<210> 59
<211> 25
<212> DNA
<213> Artificial sequence


<223>  Oligonucleotide  SV40A-2  used  for  sequencing  the
different cloning vectors used in the recombination.


<400> 59
gtgaaggaac cttacttctg tggtg                                    25



<210> 60
<211> 17
<212> DNA
<213> Artificial sequence


<223>  Oligonucleotide  Sp6  Promoter  used  for  sequencing  the
different cloning vectors used in the recombination.


<400> 60
atttaggtga cactata
17



<210> 61
<211> 20
<212> DNA
<213> Artificial sequence


<223>  Oligonucleotide  T7  Promoter  used  for  sequencing  the
different cloning vectors used in the recombination.


<400> 61
taatacgact cactataggg                                          20
```

```
<210> 62
<211> 26
<212> DNA
<213> Artificial sequence

<223> Oligonucleotide URA3-U used for cloning the URA gene of
yeasts.

<400> 62
gcccagtatt cttaacccaa actgca                                    26


<210> 63
<211> 25
<212> DNA
<213> Artificial sequence

<223> Oligonucleotide URA3-L used for cloning the URA gene of
yeasts.

<400> 63
cttccaccca tgtctctttg agcaa                                     25


<210> 64
<211> 26
<212> DNA
<213> Artificial sequence

<223> Oligonucleotide TRP1 used for detecting the auxotrophic
YAC sequences .

<400> 64
```

gcccaataga aagagaacaa ttgacc                              26

<210> 65
<211> 23
<212> DNA
<213> Artificial sequence

<223> Oligonucleotide TRP2 used for detecting the auxotrophic YAC sequences .

<400> 65
acacctccgc ttacatcaac acc
23

<210> 66
<211> 23
<212> DNA
<213> Artificial sequence

<223> Oligonucleotide LYS1 used for detecting the auxotrophic YAC sequences .

<400> 66
accaagccag catctgtatc acc
23

<210> 67
<211> 23
<212> DNA
<213> Artificial sequence

<223> Oligonucleotide LYS2 used for detecting the auxotrophic

YAC sequences .

<400> 67

gccaaatcca tccacttctc atc

23

<210> 68

<211> 20

<212> DNA

<213> Artificial sequence

<223> Oligonucleotide ProbeAPP-seq2-U used for generating the probes for the Southern blot analysis.

<400> 68

tgaatcttgg catttgggcc                                              20

<210> 69

<211> 20

<212> DNA

<213> Artificial sequence

<223> Oligonucleotide ProbeAPP-seq2-L used for generating the probes for the Southern blot analysis.

<400> 69

cctgtaactt gccctcatag                                              20

<210> 70

<211> 21

<212> DNA

<213> Artificial sequence

<223> Oligonucleotide ProbeAPP-seqA-U used for generating the probes for the Southern blot analysis.

<400> 70
gaaacgagtt gaaaggcaca g

21

<210> 71
<211> 21
<212> DNA
<213> Artificial sequence

<223> Oligonucleotide ProbeAPP-seqA-L used for generating the probes for the Southern blot analysis.

<400> 71
tttacccttc catgaagtcc c

21

<210> 72
<211> 24
<212> DNA
<213> Artificial sequence

<223> Oligonucleotide ProbeAPP-seqB-U used for generating the probes for the Southern blot analysis.

<400> 72
ggcttgtaaa ttaaactctg aagc                                    24

<210> 73
<211> 24

```
<212> DNA
<213> Artificial sequence

<223> Oligonucleotide ProbeAPP-seqB-L used for generating the
probes for the Southern blot analysis.

<400> 73
cctcagtggc tgttttgaga gcat                                    24
```

**Claims**

1. An isolated polynucleotide consisting of the nucleotide sequence comprised between nucleotide 26,064,934 and nucleotide 26,642,665 of human chromosome 21 (NCBI35:21:26064934:26642665:1), or a fragment of said polynucleotide comprising the complete human APP gene (hAPP) together with the regulatory sequences thereof .

2. Polynucleotide according to claim 1, wherein said complete hAPP gene together with the regulatory sequences thereof is comprised between nucleotide 26,174,733 and nucleotide 26,464,809 of human chromosome 21 (NCBI35: 21:26174733:26464809:1).

3. Polynucleotide according to claim 1, consisting of the nucleotide sequence comprised between nucleotide 26,064,934 and nucleotide 26,573,344 of human chromosome 21 (NCBI35:21: 26064934:26573344:1).

4. Polynucleotide according to claim 1, wherein said hAPP gene is flanked at its 5' end (in relation to the origin and the direction of the APP gene transcription) by a first nucleotide sequence and at its 3' end (in relation to the end and the direction of the APP gene transcription) by a second nucleotide sequence, wherein:

   - said first nucleotide sequence consists by the nucleotide sequence comprised between nucleotide 26,464,810 and nucleotide 26,642,665 of human chromosome 21 (NCBI35:21:26464810:26642665:1), or a fragment thereof comprising at least one unique sequence, and
   - said second nucleotide sequence consists by the nucleotide sequence comprised between nucleotide 26,064,934 and nucleotide 26,174,732 of human chromosome 21 (NCBI35:21:26064934:26174732:1), or a fragment thereof comprising at least one unique sequence.

5. Polynucleotide according to claim 4, wherein:

   - said first nucleotide sequence comprises the nucleotide sequence comprised between nucleotide 26,464,810 and nucleotide 26,573,344 of human chromosome 21 (NCBI35:21:26464810:26573344:1), or a fragment thereof comprising at least one unique sequence, and
   - said second nucleotide sequence comprises the nucleotide sequence comprised between nucleotide 26,064,934 and nucleotide 26,174,732 of human chromosome 21 (NCBI35:21:26064934:26174732:1), or a fragment thereof comprising at least one unique sequence.

6. Polynucleotide according to any of claims 4 or 5, wherein said first nucleotide sequence comprises the nucleotide sequence comprised between nucleotide 26,571,876 and nucleotide 26,573,344 of human chromosome 21 (NCBI35: 21:26571876:26573344:1).

7. Polynucleotide according to any of claims 4 or 5, wherein said second nucleotide sequence comprises the nucleotide sequence comprised between nucleotide 26,064,934 and nucleotide 26,067,221 of human chromosome 21 (NCBI35: 21:26064934:26067221:1).

8. A vector comprising a polynucleotide according to any of claims 1 to 7.

9. Vector according to claim 8, selected from the group formed by a YAC (yeast artificial chromosome), a BAC (bacterial artificial chromosome) or a PAC (P1-derived artificial chromosome).

10. Vector according to claim 8, wherein said polynucleotide is flanked at one of its ends by a third nucleotide sequence and at the other opposite end by a fourth nucleotide sequence, wherein:

    - said third nucleotide sequence comprises (i) a telomere functional in yeast cells and at least (ii) one auxotrophic selection marker, and
    - said fourth nucleotide sequence comprises (i) a centromere functional in yeast cells, a telomere functional in yeast cells and at least one auxotrophic selection marker.

11. Vector according to claim 10, wherein said auxotrophic selection marker is selected from the group comprised between *TRP1, LEU2, LYS2, HIS3, HIS5, TRP1, URA3*.

12. A cell comprising a polynucleotide according to any of claims 1 to 7, or a vector according to any of claims 8 to 11.

**13.** A transgenic non-human animal containing a polynucleotide according to any of claims 1 to 7 inserted in the genome thereof.

**14.** Transgenic animal according to claim 13, wherein said animal is a mammal, preferably a rodent, more preferably a mouse or a rat.

**15.** Transgenic animal according to claim 13 or 14, the genome of which further contains one or more molecular markers of Alzheimer's disease.

**16.** Transgenic animal according to claim 15, wherein said molecular markers of Alzheimer's disease are selected from the group comprised between the *PS1, PS2* genes and the epsilon4 ($\varepsilon$4) allele of the APOE gene.

**17.** Use of a transgenic non-human animal according to any of claims 13 to 16 as an animal model for studying Alzheimer's disease, both Sporadic Alzheimer's disease (SAD) and Familial Alzheimer's disease (FAD).

**18.** Use of a transgenic non-human animal according to any of claims 13-16 as an Alzheimer's disease model for studying the etiology of the disease, the causes, the triggering factors, the environmental and/or physiopathological aggressions or insults leading to the establishment of the disease.

**19.** Use of a transgenic non-human animal according to any of claims 13 to 16 for screening, discovering, identifying, evaluating and validating potentially useful compounds for the treatment and/or prevention of Alzheimer's disease.

**20.** Use of a transgenic non-human animal according to any of claims 13 to 16 for obtaining a transgenic non-human animal expressing only the hAPP gene.

**21.** A method for identifying a potentially useful compound for the treatment and/or prevention of Alzheimer's disease, comprising the steps of:

a) administering a candidate compound to a transgenic non-human animal according to any of claims 13 to 16;
b) determining the expression pattern of the hAPP gene and/or the hAPP protein production and/or the $\beta$-amyloid (A$\beta$) peptide production in said transgenic non-human animal of step (a), and
c) selecting the candidate compound causing (i) a decrease in the hAPP gene expression levels in nervous tissue, (ii) a decrease of hAPP protein production and/or accumulation and/or (iii) a decrease in A$\beta$ peptide production and/or accumulation in said transgenic non-human animal

**22.** The offspring of a transgenic non-human animal endogenously expressing the human APP gene with an expression pattern similar or equivalent to the expression pattern of said gene in humans.

Figure 1

Figure 2

Figure 3

Figure 4

Figure 5

Figure 6

Figure 7

Figure 9

Figure 10

Figure 11

Figure 12

Exon 6

GABPA

Figure 13

Figure 14

Figure 15

Figure 16

Figure 17

Figure 18

APP GENE

🔘 LYS 2 Arm

⬤ Seq 2 at 120kb from promoter

🔘 Seq 1 at 60kb from promoter

⬤ 17 PCRs of exons, Ex12 and 13 are in the same PCR

⬤ SeqB´at 60kb from exon 18

🔘 SeqB at 80kb from exon 18

⬤ TRP 1 Arm

Figure 19

Figure 20

Figure 21

Figure 22

Figure 23

Figure 24

Figure 25

Figure 26

Figure 27

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005098010 A

**Non-patent literature cited in the description**

- **BUXBAUM, J. D. et al.** Expression of APP in brains of transgenic mice containing the entire human APP gene. *Biochem Biophys Res Commun,* 1993, vol. 197 (2), 639-45
- **LAMB, B. T. et al.** Introduction and expression of the 400 kilobase amyloid precursor protein gene in transgenic mice [corrected]. *Nat Genet,* 1993, vol. 5 (1), 22-30
- **PEARSON, B. E. ; T. K. CHOI.** Expression of the human beta-amyloid precursor protein gene from a yeast artificial chromosome in transgenic mice. *Proc Nat1 Acad Sci USA,* 1993, vol. 90 (22), 10578-82
- **LAMB, B. T. et al.** Altered metabolism of familial Alzheimer's disease-linked amyloid precursor protein variants in yeast artificial chromosome transgenic mice. *Hum Mol Genet,* 1997, vol. 6 (9), 1535-41
- **JURKA, J.** Repbase Update: a database and an electronic journal of repetitive elements. *Trend in Genetics,* 2000, vol. 16 (9), 418-420
- **GIRALDO, P. ; MONTOLIU L.** Size matters: use of YACs, BACs and PACs in transgenic animals. *Transgenic Research,* 2001, vol. 10 (2), 83-110
- **SAMBROK et al.** Molecular cloning, a Laboratory Manual. Cold Spring Harbor Laboratory Press, 1989, vol. 1-3
- **SAMBROOCK, J. ; AUSUBEL FM, B. R. ; KINGSTON RE ; MOORE DD ; SEIDMAN JG ; SMITH JA ; STRUHL, K.** Current Protocols in Molecular Biology. John Wiley and Sons, 1989
- YAC Protocols_[Please complete the reference. **MARKIE, D.** Methods in Yeast Genetics. Cold Spring Harbor Laboratory Press, 1995

- **BECKER, D. M. ; L. GUARENTE.** High-efficiency transformation of yeast by electroporation. *Methods Enzymol,* 1991, vol. 194, 182-7
- **MOREIRA, P.N. et al.** Mouse ICSI with frozen-thawed sperm: the impact of sperm freezing procedure and sperm donor strain. *Mol Reprod Dev.,* 2003, vol. 66 (1), 98-103
- **MOREIRA, P.N. et al.** Efficient generation of transgenic mice with yeast artificial chromosomes by intracytoplasmic sperm injection. *Biol Reprod.,* 2004, vol. 71 (6), 1943-7
- **LEWIS, B. C. ; N. P. SHAH ; B. S. BRAUN ; C. T. DENNY.** Creation of a yeast artificial chromosome fragmentation vector based on lysine-2. *Genet Anal Tech Appl,* 1992, vol. 9 (3), 86-90
- **BURKE, D. T. ; G. F. CARLE ; M. V. OLSON.** Cloning of large segments of exogenous DNA into yeast by means of artificial chromosome vectors. 1987. *Biotechnology,* 1992, vol. 24, 172-8
- **BURKE, D. T. ; M. V. OLSON.** Preparation of clone libraries in yeast artificial-chromosome vectors. *Methods Enzymol,* 1991, vol. 194, 251-70
- **ALANI, E., L. CAO ; N. KLECKNER.** A method for gene disruption that allows repeated use of URA3 selection in the construction of multiply disrupted yeast strains. *Genetics,* 1987, vol. 116 (4), 541-5
- **LEHMAN, E. J. et al.** Genetic background regulates beta-amyloid precursor protein processing and beta-amyloid deposition in the mouse. *Hum Mol Genet,* 2003, vol. 12 (22), 2949-56